(19) Europäisches Patentamt
European Patent Office
Office européen des brevets

(11) EP 2 758 772 B1

# (12) EUROPEAN PATENT SPECIFICATION

(45) Date of publication and mention of the grant of the patent:
**24.10.2018 Bulletin 2018/43**

(21) Application number: **12859654.1**

(22) Date of filing: **19.09.2012**

(51) Int Cl.:
***G01N 27/12*** *(2006.01)*

(86) International application number:
**PCT/US2012/056096**

(87) International publication number:
**WO 2013/095730 (27.06.2013 Gazette 2013/26)**

## (54) MULTIMODE PLATFORM FOR DETECTION OF COMPOUNDS

MULTIMODALE PLATTFORM FÜR DEN NACHWEIS VON VERBINDUNGEN

PLATEFORME MULTIMODE POUR LA DÉTECTION DE COMPOSÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2011 US 201161626011 P**

(43) Date of publication of application:
**30.07.2014 Bulletin 2014/31**

(73) Proprietor: **University of Utah Research Foundation**
**Salt Lake City, UT 84108 (US)**

(72) Inventors:
- **ZANG, Ling**
  **Salt Lake City, UT 84108 (US)**
- **BUNES, Benjamin**
  **Salt Lake City, UT 84103 (US)**
- **XU, Miao**
  **Salt Lake City, UT 84108 (US)**

(74) Representative: **Walker, Ross Thomson**
**Forresters IP LLP**
**Skygarden**
**Erika-Mann-Strasse 11**
**80636 München (DE)**

(56) References cited:
**WO-A2-2011/079296**
**WO-A2-2011/119752**
**CN-A- 101 907 593**
**KR-A- 20070 097 775**
**US-A1- 2006 078 468**
**US-A1- 2006 263 255**
**US-A1- 2007 158 209**
**US-A1- 2008 150 556**
**US-A1- 2009 233 374**

- **NADDO T ET AL: "Highly responsive fluorescent sensing of explosives taggant with an organic nanofibril film", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 134, no. 1, 8 May 2008 (2008-05-08), pages 287-291, XP023979359, ISSN: 0925-4005, DOI: 10.1016/J.SNB.2008.05.001 [retrieved on 2008-05-08]**
- **CHENGYI ZHANG ET AL: "Organic nanofibrils based on linear carbazole trimer for explosive sensing", CHEMICAL COMMUNICATIONS, vol. 46, no. 30, 24 June 2010 (2010-06-24) , pages 5560-5562, XP055188253, ISSN: 1359-7345, DOI: 10.1039/c0cc01258k**
- **SHENGYANG TAO ET AL: "Fluorescent nanofibrous membranes for trace detection of TNT vapor", JOURNAL OF MATERIALS CHEMISTRY, vol. 17, no. 26, 25 April 2007 (2007-04-25), page 2730, XP055071374, ISSN: 0959-9428, DOI: 10.1039/b618122h**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 758 772 B1

**Description**

**FIELD OF THE INVENTION**

This invention relates generally to materials, chemical sensors and detector devices. Therefore, the present invention relates generally to the fields of chemistry, materials science, chemical engineering, and electrical engineering.

**BACKGROUND**

Various vapor sensing devices have been employed to provide a means for monitoring and controlling organic compounds, particularly illicit and security threatening agents like drugs and explosives. Such devices can include chemiresistors and semiconductor devices. Compared to inorganic chemiresistors, organic chemiresistors offer facile deposition procedure as well as various choices and easy tuning of receptors for analyte molecules. Although organic field-effect transistors (FETs) can also be used to detect chemical species, the fabrication is relatively complicated and the performance is affected by many factors, like grain boundaries, surface morphology, molecular structure, etc. As such, novel sensor devices continue to be sought through ongoing development and research efforts.

US 2008/150556 discloses a gas sensor platform comprising an array of electrode pairs on a substrate, each electrode pair having organic nanofibers deposited thereon, said nanofibers varying their electrical impedance upon exposure to specific target materials.

NADDO T et al in SENSORS AND ACTUATORS B, vol. 134, no. 1, 8 May 2008 (2008-05-08), pages 287-291 relates to "Highly responsive fluorescent sensing of explosives taggant with an organic nanofibril film".

WO2011/119752 discloses a gas sensor comprising a pair of electrodes and a nanofiber mass deposited thereon, the nanofiber mass varying its electrical conductivity upon exposure to a specific target material and excitation light.

**SUMMARY**

In a first aspect of the present invention there is provided a multimode gas sensor platform, comprising: an array of electrode pairs oriented on a substrate, wherein individual electrode pairs are separately addressable; and a plurality of detection zones, each detection zone comprising at least one set of individual electrode pairs within the array, said at least one set of individual electrode pairs having organic nanofibers uniformly deposited thereon, said organic nanofibers being responsive to association with a corresponding target material such that the associated nanofibers vary their electrical conductivity upon exposure to specific target materials and a source excitation light, and wherein each detection zone has a different nanofiber material; and further comprising a housing having an inlet and an outlet, wherein the plurality of detection zones are positioned in the housing between the inlet and the outlet; and a light source configured to illuminate at least a first detection zone within the plurality of detection zones.

In a further aspect of the present invention there is provided a method of detecting an explosive comprising exposing the multimode platform of the present invention to a target sample and measuring electrical responses of the organic nanofibers.

**BRIEF DESCRIPTION OF THE DRAWINGS**

Features and advantages of the invention will be apparent from the detailed description which follows, taken in conjunction with the accompanying drawings, which together illustrate, by way of example, features of the invention; and, wherein:

FIG. 1 shows a perspective schematic view of a multimode gas sensor platform in accordance with one embodiment of the present invention;

FIG. 2 shows a cross-sectional view of a sensor in accordance with one embodiment of the present invention;

FIGs. 3A-F show cross-sectional views of a multimode gas sensor platform during placement of organic nanofibers;

FIG. 4 is a flow chart of a deposition method;

FIG. 5 shows a cross-sectional view of a multimode gas sensor platform;

FIG. 6 is a flow chart of a method for attaching ligands;

FIG. 7 is a flow chart of a method in accordance with one embodiment of the present invention;

FIGs. 8A-B shows a photograph of organic nanofibers in a glass cylinder (A) and the resultant nanofiber mesh (B) compound;

FIG. 9 shows optical and fluorescence images of nanofibers deposited on untreated substrate (left) and OTS-treated substrates (right);

FIG. 10 is a plot of relative response vs. time for a target compound;

FIG. 11 is a bar graph of relative responses of three compounds for three different sensor materials in accordance with one embodiment of the present invention;

FIG. 12 is a flow chart for determining a target compound in accordance with one embodiment of the present invention; and

FIG. 13 shows plots of the relative responses for two sensors to two analytes in accordance with one embodiment of the present invention.

These figures are not necessarily to scale and actual dimensions may, and likely will, deviate from those represented. Thus, the drawings should be considered illustrative of various aspects of the invention while not being limiting. Reference will now be made to the exemplary embodiments illustrated, and specific language will be used herein to describe the same. It will nevertheless be understood that no limitation of the scope of the invention is thereby intended.

## DETAILED DESCRIPTION OF EXAMPLE EMBODIMENTS

Before the present invention is disclosed and described, it is to be understood that this disclosure is not limited to the particular process steps and materials disclosed herein because such process steps and materials may vary somewhat. It is also to be understood that the terminology used herein is used for the purpose of describing particular embodiments only. The terms are not intended to be limiting because the scope of the present disclosure is intended to be limited only by the appended claims.

It must be noted that, as used in this specification and the appended claims, the singular forms "a," "an," and "the" include plural referents unless the context clearly dictates otherwise.

As used herein, "alkyl" refers to a branched, unbranched, or cyclic saturated hydrocarbon group, which typically, although not necessarily, contains from 1 to about 50 carbon atoms, or 1 to about 40 carbon atoms, or 1 to about 30 carbon atoms for example. Alkyls include, but are not limited to, methyl, ethyl, n-propyl, isopropyl, n-butyl, isobutyl, t-butyl, octyl, and decyl, for example, as well as cycloalkyl groups such as cyclopentyl, and cyclohexyl, for example. As used herein, "substituted alkyl" refers to an alkyl substituted with one or more substituent groups. The term "functionalized alkyl" refers to alkyls having functional groups including, but not limited to, heteroatoms, e.g., oxygen and nitrogen; carbonyls; aromatic groups; multiple bonds; and ionic groups. Such functionalized alkyls can therefore include esters, ethers, aryls, ketones, aldehydes, carboxylic acids, alcohols, amines, amides, salts, etc. The term "heteroalkyl" refers to an alkyl in which at least one carbon atom is replaced with a heteroatom. If not otherwise indicated, the term "alkyl" includes unsubstituted alkyl, substituted alkyl, lower alkyl, functionalized alkyls, and heteroalkyl.

As used herein, "nanofiber" refers to any elongated structure having a nanoscale cross-section such as, but not limited to, nanowires, nanobelts, nanoribbons, or other nanofibrous materials.

As used herein, "explosive" refers to explosive compounds, explosive byproducts, and explosive precursors, unless the context dictates otherwise.

As used herein, the term "about" is used to provide flexibility to a numerical range endpoint by providing that a given value may be "a little above" or "a little below" the endpoint. The degree of flexibility of this term can be dictated by the particular variable and would be within the knowledge of those skilled in the art to determine based on experience and the associated description herein.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Concentrations, amounts, and other numerical data may be expressed or presented herein in a range format. It is to be understood that such a range format is used merely for convenience and brevity and thus should be interpreted

flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 wt% to about 5 wt%" should be interpreted to include not only the explicitly recited values of about 1 wt% to about 5 wt%, but also include individual values and sub-ranges within the indicated range. Thus, included in this numerical range are individual values such as 2, 3.5, and 4 and sub-ranges such as from 1-3, from 2-4, and from 3-5, etc. This same principle applies to ranges reciting only one numerical value. Furthermore, such an interpretation should apply regardless of the breadth of the range or the characteristics being described.

The present inventors have discovered the effective use and construction of a multimode gas sensor platform using uniformly assembled organic nanofibers for detection of various compounds using electrically responsive detection. Additionally, the present inventors have discovered novel sensors and methods related thereto. Notably, such platforms and sensors can be used in the selective detection of target compounds, including explosives.

The corresponding target materials can include any compound that provides an electronic response when exposed to an organic nanofiber. Each detection zone can be selective to a different target compound. In one embodiment, the corresponding target material can be one or more of explosive compounds, explosive byproducts, and explosive precursors. In another embodiment, the corresponding target material can be drugs and narcotics, toxic compounds, industrial compounds, and mixtures thereof.

Drugs can include, but are not limited to, narcotic or hallucinogen compounds, byproducts thereof, or precursors thereof. Non-limiting examples can include methamphetamine, heroin, cocaine, and composites or combinations thereof. Non-limiting examples of toxic compounds can include hydrazine and hydrogen sulfide. Non-limiting examples of industrial compounds include ammonia, ammonium nitrate, and chlorine.

Explosives can generally be nitro-based explosives, ammonia and inorganic salt-based explosives, peroxide-based explosives, byproducts thereof, precursors thereof, and mixtures of these explosives. In one aspect, the explosive can be nitro-based and can be selected from the group consisting of trinitrotoluene (TNT); dinitrotoluene (DNT); 2,3-dimethyl-2,3-dinitrobutane (DMNB); 1,3,5-trinitroperhydro-1,3,5-triazine (RDX); pentaerythritol tetranitrate (PETN); octahydro-1,3,5,7-tetranitro-1,3,5,7-tetrazocine (HMX); nitromethane; nitroglycerin; nitrocellulose; ethylene glycol dinitrate; ammonium nitrate; urea nitrate; and the like. RDX and PETN have extremely low vapor pressure which makes them difficult to detect using conventional technologies.

In another embodiment, the explosive can be ammonia or inorganic salt-based such as, but not limited to, ammonia nitrate ($NH_4NO_3$), urea nitrate ($(NH_2)_2COHNO_3$), and highly oxidative reagents such as potassium nitrate ($KNO_3$), potassium chlorate ($KClO_3$), and potassium perchlorate ($KClO_4$), dimethyl methylphosphonate, and combinations of these explosives.

Non-limiting examples of suitable peroxide-based explosives can include acetone peroxides; triacetone triperoxide (TATP); peroxyacetone; tri-cyclic acetone peroxide (TCAP); diacetone diperoxide (DADP); hexamethylene triperoxide diamine (HMTD); and composites or combinations thereof. Other explosives and explosive additives can also be detected.

The detection zones described herein generally include a pair of electrodes and organic nanofibers deposited thereon. In one embodiment, the individual electrode pairs can be interdigitated electrodes. In one embodiment, each of the detection zones can be configured to detect an explosive compound. Detection can be made by changes in electrical signals across the electrode pair. Specifically, in accordance with the present invention, the associated nanofibers are chosen to vary their electrical conductivity upon exposure to specific target materials and to a source excitation light. In this manner, each pair of electrodes can be associated with specific nanofibers which are selectively responsive.

Each detection zone can comprise individual electrode pairs with unique nanofibers on each pair. Alternatively, multiple electrode pairs can be associated with a common nanofiber type so as to provide multiple signal sources for a particular target material. These common detection blocks can be grouped adjacent one another or distributed across the array among other detection zones which have different nanofiber materials. As such, the detection zones can include a plurality of electrode pairs. In one embodiment, each detection zone can be configured for sensing a different compound. In another embodiment, each detection zone can be configured for sensing a group of compounds. In one embodiment, the group of compounds can be grouped based on their structure, e.g., nitro-based compound. In another embodiment, the group of compounds can be grouped based on their function, e.g., explosive compounds.

In addition to the electrical responses, at least one detection zone can be configured for an optical or visual response. In one embodiment, the plurality of detection zones can include at least one visual detection zone comprising organic nanofibers that fluoresce or have a visual color change when exposed to an explosive compound, an explosive byproduct, or an explosive precursor.

The organic nanofibers discussed herein include any nanofibers that provide an electronic response when exposed to a target compound, although optically responsive nanofibers can also be additionally used. Generally, the organic nanofibers are deposited onto the electrode pair such that the mass of nanofibers maintains a porosity sufficient

to allow a target sample to penetrate therein and provide an electronic. Optimal or desired porosity can vary depending on a variety of factors such as, but not limited to, nanofiber type, desired sensitivity, available surface area, target material, and the like. However, typically a pore size ranging from a several nanometers to a several hundreds of nanometers can be suitable, e.g. 5 nm to 400 nm.

Generally, the nanofibers are present on the electrode pair as a nanofiber mass. In one embodiment, the nanofiber mass can be a film. In one embodiment, the film can be highly porous. In one embodiment, the film can have a thickness ranging from 20 nm to 10 $\mu$m. As such, the organic nanofibers described herein can form a porous film of entangled nanofibers. In one embodiment, the organic nanofibers can have a diameter of about 10 nm to about 900 nm. Although not always required, the organic nanofibers used herein can most often be formed as cofacially stacked fibers where individual constituent molecules are stacked co-facially to form the nanofiber. These structures benefit from pi-pi interactions and bonding between adjacent molecules along the nanofiber. Furthermore, although the nanofibers can be modified (e.g., surface coverage with electron donor or acceptor to enhance the interface charge separation), typically, at least some detection zones utilize nanofibers which are structurally unmodified from the as-formed nanofiber material.

Typically, the building block molecules of the organic nanofibers can be individually selected from the group consisting of: carbazole-cornered, arylene-ethynylene tetracyclic macromolecules, indolocarbazole derivatives thereof, a substituted perylene tetracarboxylic diimide molecule, a substituted a 3,4,9,10-tetracarboxyl perylene molecule, and mixtures thereof. Each of these nanofibers can be more or less selective for particular target explosive compounds. As target compounds are encountered, the target compound is associated with the nanofiber material. The nature of association is not completely understood in all cases. However, the association can be hydrogen bonding, intermediate formation, pi-pi stacking, Van der Waals force, hydrophilic/hydrophobic forces, metal ion/ligand coordination complex, and the like. For example, carbazole-cornered nanofibers can be particularly effective at bonding with nitro-based explosives (e.g. TNT, DNT, nitromethane, DMNB, RDX, PETN, etc.). Arylene-ethynylene tetracyclic macromolecules can be effective at detecting nitro-based explosives. Similarly, indolocarbazole derivatives thereof can be sensitive to detecting nitro-based explosives. In contrast, substituted perylene tetracarboxylic diimide molecule can be particularly suited to detection of ammonia, amine-containing compounds, and fertilizer based explosives such as ammonia nitrate ($NH_4NO_3$), urea nitrate ($(NH_2)_2COHNO_3$), and narcotic or hallucinogen compounds, e.g. methamphetamine, heroin, cocaine, etc., which contain amine groups. A substituted a 3,4,9,10-tetracarboxyl perylene molecule can also be suitable for detection of electron deficient or withdrawing compounds, like nitro-based explosives (e.g. TNT, DNT, nitromethane, DMNB, RDX, and PETN). The mechanism for detection using such perylene molecule based nanofibers is via depletion of photogenerated electrons flowing through the nanofibers.

In one embodiment, the organic nanofibers can be individually selected from the group consisting of: a 3,4,9,10-tetracarboxyl perylene compound having structure I:

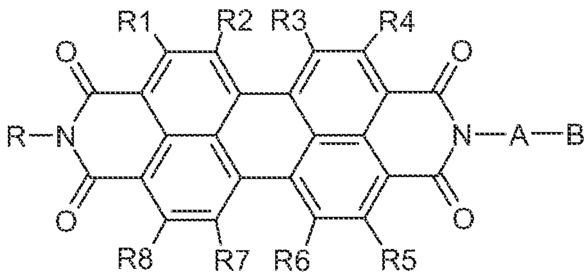

(I)

where R is a morphology control group, A is a linking group, B is a electron donor that is selective for transferring electrons to PTCDI backbone upon irradiation to make the resulting nanostructures conductive, and R1 through R8 are side groups; a alkyl-substituted, carbazole-cornered, arylene-ethynylene tetracyclic macromolecule of formula II:

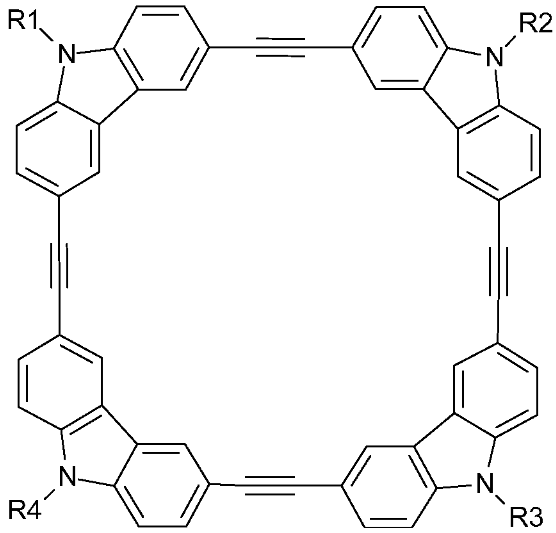

Formula II

wherein R1-R4 are alkyl groups and wherein at least some of the macromolecules are cofacially stacked; and mixtures thereof. Notably, such structures are described in WO 2011/079296 and WO 2011/119752.

In addition to the electrically responsive materials discussed herein, the present nanofibers can include those that can provide a visual response. As such, in one embodiment, the organic nanofibers can be selected from the group consisting of: a linear carbazole oligomer, a porous hydrophilic material modified with a titanium oxo compound, and mixtures thereof.

In one embodiment, the organic nanofibers can be individually selected from the group consisting of: a 3,4,9,10-tetracarboxyl perylene compound having the structure of formula III:

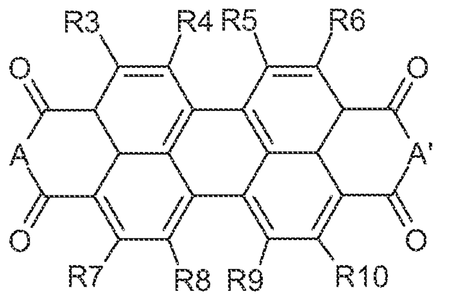

(III)

where A and A' are independently chosen from N-R1, N-R2, and O such that both A and A' are not O, and R1 through R10 are amine binding moieties, solubility enhancing groups, or hydrogen such that at least one of R1 through R10 is an amine binding moiety; a linear carbazole oligomer having the structure of formula IV:

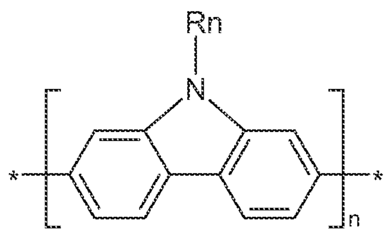

Formula IV

where n is 3 to 9, Rn are independently selected amine side groups, and at least one Rn is a C1 to C14 alkyl; a carbazole-cornered, arylene-ethynylene tetracyclic macromolecules of formula V:

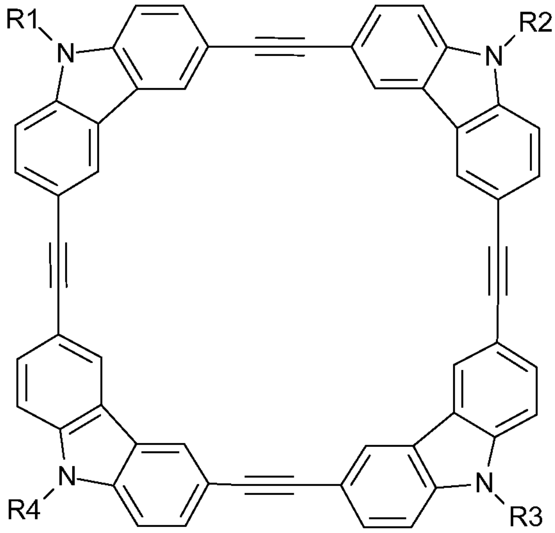

Formula V

wherein R1-R4 are alkyl-containing groups that facilitate cofacial stacking to form tubular morphology, and wherein at least some of the macromolecules are cofacially stacked; a porous hydrophilic material modified with a titanium oxo compound having the structure of formula VI:

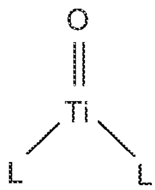

Formula VI

where L is a ligand, wherein the porous hydrophilic material is capable of detecting hydrogen peroxide vapor by complexing the titanium oxo compound with the hydrogen peroxide to provide a color change; and mixtures thereof. Notably, such structures are described in US 2010/0197039, WO 2012/047330, co-pending WO2013/066458 and WO 2011/100010.

A sensor for detecting explosives comprises a housing having an inlet and an outlet, any multimode platform described herein positioned in the housing between the inlet and the outlet, and a light source configured to illuminate at least a first detection zone within the plurality of detection zones. The housing can also optionally be configured to interface or connect with a handheld computer or other device.

Generally, the sensor is configured to measure the electronic response from the detection zones. Additionally, the detection zones are individually addressable including, but not necessarily, having individual light sources configured to illuminate corresponding detection zones. Light sources can be dedicated to illuminate a single electrode pair or can illuminate multiple electrode pairs within a region of the array. As such, in one embodiment, the sensor can comprise a second light source that is configured to illuminate a second detection zone within the plurality of detection zones. The light sources discussed herein can be any light source capable of illuminating a detection zone, and in one aspect, individual discrete detections zones. As such, in one embodiment, the light source can be an LED. Light having a wavelength from about 250 nm to about 800 nm can be suitable. Although the light sources can be continuous, the light sources can optionally be configured for intermittent power. Specifically, the light sources can be cycled to turn on at periodic intervals, i.e. every second, every ten seconds, etc. Generally, frequency of illumination can range from about 1 millisecond to about 600 seconds. The pulse duration for each illumination cycle can also be varied. The desired pulse duration can vary depending on the characteristic response properties of the nanofibers and associated target materials. However, as a general guideline, pulse durations can range from about 1 millisecond to about 600 seconds. Furthermore, detections zones can be selectively activated in either a continuous or pulsed illumination scheme. For example, a peroxide detection zone can be activated while leaving other zones inactive.

Additionally, as discussed herein, the plurality of detection zones can include at least one visual detection zone comprising organic nanofibers that fluoresce or have a visual color change when exposed to the corresponding target material. Further, the sensor can comprise a photodetector configured to detect the fluorescence or visual color change.

The sensor can be configured to move a target compound, in one embodiment a sample of air containing the target compound, through the sensor to allow for detection. As such, in one embodiment, the substrate of the multimode

platform in the sensor can include a plurality of holes allowing air flow from a top surface of the substrate to a bottom surface of the substrate. Additionally, the sensor can include a forced air mechanism adapted to move air across at least a portion of the plurality of detection zones. This forced air mechanism can be a low profile, low amperage fan, pump, or any other mechanism which directs air across the detection zones and array of electrode pairs. This allows any target material within the sample air to contact the detection zones.

The multimode platform can be isolated within the housing such that the air flow is forced across the detection zones and optionally through the plurality of holes. Generally, the substrate can be manufactured of any material that can support the electrode pairs. In one embodiment, the substrate can be silicon. The electrode pairs can have widths and inter-electrode gap distances which are adapted or optimized for the corresponding nanofiber deposited on that electrode pair. The electrodes can be formed of any suitable conductive material. Non-limiting examples of suitable conductive material includes gold, copper, silver, aluminum, conductive polymers, and the like. Although electrode lengths (i.e. gaps) can be varied, inter-electrode gaps can be a stronger factor in optimizing signals. Generally, inter-electrode gap distances can range from about 10 nm to about 100 $\mu$m. The gap distance can be driven by the resistivity of the materials. For example, a highly conductive material may benefit from a relatively large gap because it increases the effective area over which a target molecule can be captured (i.e., more likely to capture a molecule than one with a shorter gap). Conversely, the resistivity sets an upper limit for how large this gap can be. As such, a gap size can balance the capture zone with the resistivity of the nanofibers. Electrode widths can also be varied. In the context of interdigitated electrodes, widths are defined as electrode finger lengths times the number of fingers within an interdigitated electrode pair. For example, widths can range from about 20 nm (e.g. a single fiber) to 1 m (large, interdigitated electrodes). Variations in width can be affected by similar factors as electrode gaps. Each of these dimensions can be adjusted to balance signal and fit into a specific form factor for a given configuration. Further, each electrode pair can have a top plan surface area with dimensions from about 1 mm to about 50 mm, and in some cases 2 mm to about 10 mm in width and length. However, in one aspect, the top plan surface area can be about 5 mm square. Although specific electrode dimensions can vary, it can be desirable to adjust the electrode dimensions in order to provide a target maximum resistance across the electrodes. For example, in some configurations, it can be desirable to limit maximum resistance to about 1 M$\Omega$, although a maximum of about 10 G$\Omega$ can also be useful.

Each of the electrode pairs can include electrical traces which individually connect to data collection and/or processing modules. Thus, changes in signals can be associated with each electrode pair independent of other electrode pairs within the array.

The sensor can further comprise a microcontroller module adapted to measure a binding profile of a test sample and to correlate the binding profile with predetermined target compound binding profiles. As discussed herein, such binding profiles can correspond to one or more response characteristics of the sensor to exposure to specific target compounds including: change in resistance, rate of response, rate of recovery, reversibility, emission change and the like. Such characteristics can correspond to the change in electronic responses of the sensor, including the initial amount of response measured upon exposure, residual response after exposure, the rates of change, etc. As such, the sensor can further comprise an electrical interface adapted to connect to a computing device and transmit data from the plurality of detection zones to the computing device. Such data can be used to individually identify a target compound or plurality of target compounds as discussed herein.

The sensor can include a printed circuit board having a microprocessor and memory storage to store data. In one embodiment, the computing device can be a hand held device or a computer having a visual output for indicating explosive detection.

The present sensors can have excellent sensitivity and selectivity, depending on the particular nanofiber composition and configuration. Accordingly, in one embodiment, the sensor can detect the compound in a concentration as low as 1 ppm. In one aspect, the sensor can detect the compound in a concentration as low as 1 ppb. In another aspect, the sensor can detect compound in a concentration as low as 1 ppt.

Turning now to FIG. 1, a multimode gas sensor platform 100 comprises a plurality of detection zones 104 oriented on a substrate 102. Each detection zone comprises at least one electrode pair 106 having organic nanofibers 108 uniformly deposited thereon. Uniform deposition covers uniformity in thickness of the nanofiber mass and number of nanofibers deposited across the electrode pair. Uniformity can be sufficient to obtain a statistically meaningful signal from the electrode pair upon binding with a target analyte. As discussed herein, the substrate can comprise any material which provides structural support for the electrode pairs and associated leads. Although silicon wafers and semiconductors can be desirable, other non-limiting examples of substrate materials can include glass, plastics, biological materials, ceramics, composites thereof or the like.

Turning now to FIG. 2, a sensor 200 includes a support substrate 202 positioned between an inlet 204 and outlet 206 of a housing 208. The support substrate can optionally include a plurality of holes 210 to allow air flow between the inlet and outlet. However, the support substrate can also form a planar array across which the air flows laterally. The array of electrode pairs can typically be provided as a planar array. However, individual detection zones and associated electrode pairs can be spatially oriented in various configurations. For example, the array can be formed along multiple

stacked spaced tiers, spaced substrates facing one another, about interior surfaces of a housing facing inward, or the like. Further, individual electrode pairs can be formed on an electrode substrate (e.g. silicon or other suitable material). The electrode substrate can then be bonded with the support substrate 202 along with alignment of contact pads and/or other electrical connections.

Additionally, the sensor can include an optional air flow mechanism 212 to circulate air across the detection zones 214. While positioned near the outlet 206 or inlet 204 in FIG. 2, such positioning is not intended to be limiting. The air flow mechanism can be position anywhere within the sensor or even on the outside of the housing. The substrate generally includes detection zones 214 positioned in proximity to a light source 216 and an optional photodetector 218. The sensor can optionally include a microcontroller module 220 adapted to measure a bonding profile of a test sample and to correlate the bonding profile with predetermined target compound bonding profiles. The microcontroller can generally be present in a separate module which is connected to an associated computing device which receives data from the sensor. As such, the sensor can include an electrical interface 222 adapted to connect to a computing device (not shown) and transmit data from the plurality of detection zones to the computing device. Any suitable connection can be used such as, but not limited to, USB, wireless, LAN, or any other data transfer connector or protocol.

Generally, fabrication of the sensor array relies upon the ability to deposit fibers evenly on the electrodes and confinement of each type of fiber to a specific electrode pair. One method of accomplishing this is to use wells placed on top of the electrode array. Solutions including the nanofibers are dispensed into the wells. Heat can be applied, which facilitates solvent evaporation and the formation of the nanofiber mesh. Eventually, the solvent evaporates completely, leaving a fairly uniform nanofiber mesh on the electrodes. The remaining nanofiber mass can be adherently bonded to the electrode pairs. The nanofiber mass can connect electronically to the electrode pairs.

Turning now to FIGs. 3A-F, the figures provide cross-sectional views of a manufacturing of a multimode gas sensor platform. FIG. 3A shows a substrate 302 with electrode pairs 304 attached thereto. FIG. 3B shows a distribution apparatus 306 having a plurality of wells 308 positioned over the electrode pairs. The distribution apparatus can optionally include a single well for production of individual sensor pads (i.e. an electrode substrate, a single electrode pair, and deposited nanofiber mass). Individual sensor pads can then be bonded to a corresponding support substrate (e.g. substrate 202 in FIG. 2). FIG. 3C shows the wells having a liquid carrier 310 with organic nanofibers 312 deposited therein. FIG. 3D shows the organic nanofibers settling into a mass 314. FIG. 3E shows the removal of the liquid carrier and the mass of nanofibers deposited on the electrode pairs. FIG. 3F shows the multimode gas sensor platform 316 after optional removal of the deposition apparatus.

Turning to FIG. 4, a method of uniformly depositing organic nanofibers onto an array of electrode pairs can comprise placing a distribution apparatus over the array 402, the distribution apparatus having a plurality of wells, wherein at least some of the wells are positioned over corresponding electrode pairs within the array. The method can further include depositing organic nanofibers within the wells 404. The method can also include allowing the organic nanofibers to deposit onto the electrode pairs 406. The method can also include removing the distribution apparatus 408. As with the process described above, the distribution apparatus can optionally include a single well such that a nanofiber mass is deposited on individual sensor pads which are then subsequently bonded to a support substrate.

The method can further comprise dispersing the organic nanofibers within a liquid carrier prior to depositing the nanofibers with the wells. Additionally, the method can further comprise removing the liquid carrier prior to removing the distribution apparatus. In one aspect, heat can be applied to the wells after depositing the nanofibers and the liquid carrier to help facilitate removal of the carrier fluid.

Turning now to FIG. 5, an alternate structure is shown for achieving uniform depositions of organic nanofibers onto an electrode pair. In one embodiment, the substrate 502 can be functionalized with a hydrophobic ligand 504, including between electrode pairs 506. Once the ligand is bonded to the substrate, the organic nanofibers 508 can be dispersed over the substrate where the hydrophobic ligands can interact with the nanofibers facilitating uniform deposition through hydrophobic-hydrophobic interactions.

As such, turning to FIG. 6, a method of uniformly depositing organic nanofibers onto an array of microelectrode pairs can comprise attaching hydrophobic organic ligands to exposed surfaces between corresponding microelectrodes within each microelectrode pair 602 and exposing the hydrophobic organic ligands to the organic nanofibers sufficient to allow interaction between the hydrophobic organic ligands and the organic nanofibers 604.

Generally, the hydrophobic organic ligands can be any ligands that allow for hydrophobic interactions between the ligand and an organic nanofiber. In one embodiment, the hydrophobic organic ligand can be an alkylsilane. In one aspect, the alkylsilane can be an alkyltrichlorosilane. In another embodiment, the hydrophobic organic ligands can be selected from the group consisting of: octadecyltrichiorosilane, dodecyltrichlorosilane, trichloro(hexyl)silane, trichloro(phenethyl)silane, trichloro(phenyl)silane, trichloro(1*H*,1*H*,2*H*,2*H*-perfluorooctyl)silane, trichloro(3,3,3-trifluoropropyl)silane, trichloro(octyl)silane, 1H,1H,2H,2H-perfluorooctylthethoxysilane, trichloro(phenethyl)silane, and mixtures thereof. Such surface treatment can also be used and applied with the distribution apparatus previously discussed.

Turning to FIG. 7, a method of detecting an explosive can comprise exposing a multimode platform, including any of those as described herein, to a target sample 702 and measuring electrical responses of the organic nanofibers

704. Although data processing can be accomplished in a variety of ways, the following discussion provides exemplary and useful approaches. In one embodiment, sensor responses can be measured, digitized and stored in an onboard microcontroller which accompanies the sensor array within the housing. Data are transmitted from the microcontroller to a computing device (e.g., by wireless, LAN, USB, etc.). The received signals are analyzed by the computing device to determine the values of the parameters of interest (e.g., $R_{resp}$, $R_{recov}$, $S_{resp}$, $S_{recov}$, etc.). A processing algorithm (e.g., decision tree, genetic algorithm, neural network, parameterization) can be invoked to match the measured data to a library of sensor responses to various target analytes. Matches and results can then be relayed to the user via a suitable display.

In an alternative processing approach, sensor states and responses can be measured, digitized and stored in an onboard microcontroller. The data can be analyzed by the microcontroller to determine the values of the parameters of interest (e.g., $R_{resp}$, $R_{recov}$, $S_{resp}$, and $S_{recov}$). In this case, the microcontroller can include a microcontroller and computing module to calculate values. The microcontroller can then apply the parameter values to an algorithm (e.g., decision tree, genetic algorithm, neural network, parameterization) to match the measured data to a library of sensor responses to various targets. The absence or presence of matches and any other relevant data (e.g. concentration) can be transmitted to the computer (e.g., by wireless, LAN, USB, etc.). The results can then be relayed to the user, e.g. typically via a graphical interface or display. Accordingly, the processing of data can be accomplished using a separate computing device (e.g. handheld computer, mobile device or desktop) or using electronics integrated on a circuit board to which the sensor array plugs are connected.

The present methods can optionally further comprise recycling the organic nanofibers by dissolving the nanofibers in a first organic solvent and extracting the nanofibers with a second organic solvent. In one embodiment, the first organic solvent can be a halogen-containing solvent. In one aspect, the first organic solvent can be chloroform. In another embodiment, the second organic solvent can be an aqueous alcohol mixture. In one embodiment, the second organic solvent can be a mixture of ethanol and water.

## EXAMPLES

Example 1 - *Synthesis and Fabrication of Organic Nanofiber Mass*

Glass cylinders of equal size were made by breaking the ends off glass pipets. These glass cylinders were then attached to a glass slides using a mixture of polystyrene and toluene. After the polystyrene dried, a homogenous mixture of nanofiber and solvent was added equally to each of the cylinders. The glass slides with cylinders attached were then placed on a hot plate and heated in the range of 60-70 °C. With the addition of heat, the nanofiber began to clump in the cylinders (FIG. 8A). The solvent was evaporated off until the clump fell directly onto the surface of the glass slide yielding a fairly uniform deposition (FIG. 8B). It was found that the size of the clump of nanofibers could be directly controlled by adjusting the fiber to solvent ratio of the homogenous solution.

Possible modifications to this process include changing the cross sectional shape of the wells to closely match those of the substrates, applying a coating to the interior of the wells, changing the materials used for the wells, and controlling the temperature of the walls of the wells. The bases of the wells can be sealed (for example, using a gasket and pressure) without the use of adhesive. This can allow arrays of wells to be fabricated and used multiple times in a production environment.

Example 2 - *Surface Treatment for Organic Nanofiber Deposition*

Employing a surface treatment can be used to enhance the aforementioned deposition technique, or a deposition technique not specifically described herein. A non-limiting example of this is formation of an octadecyltrichlorosilane (OTS) monolayer on the $SiO_2$ substrate (a structure similar to that as shown in FIG. 5). This was performed by soaking the substrate in an OTS/toluene solution overnight. The substrates were cleaned by subsequent sonication (30 s each) with toluene, acetone, methanol, and isopropyl alcohol. Finally, the substrates were baked to remove any remaining solvent. The hydrophobicity of the OTS-treated surface is more attractive to the nanofibers than the hydrophilic untreated surface. This surface modification was tested by drop casting a homogeneous nanofiber solution onto heated (ca. 70°C) $SiO_2$ substrates, both treated and untreated. While the untreated substrate tended to force the nanofibers to the edges (FIG. 9A), the OTS monolayer facilitated the formation of a relatively uniform film (FIG. 9B).

Example 3 - *Selective Identification of Unknown Analyte*

The present sensors array can be used in conjunction with a signal processing system to improve selectivity and reduce the rate of false positives. Such a system can monitor the responses for each sensor, including, but not limited to, change in resistance ($R_{resp}$), rate of response ($S_{resp}$), rate of recovery ($S_{recov}$), and reversibility ($R_{recov}$) (FIG.

10). Additionally, an algorithm to use the relative responses from each sensor can be employed to further enhance selectivity. Since each sensor material used in the array can have a unique response to each analyte, these responses can be leveraged in one of several ways to reduce the frequency of false positives.

In one embodiment, the algorithm can be decision tree learning. A decision tree is a step-by-step comparison of the relative responses of each sensor in the array. Since each sensor can have a unique response to each analyte (FIG. 11), the relative responses can be used to isolate the detected species to a narrow set of possibilities, potentially resolving a single chemical species. The decision tree asks a series of binary questions that narrows down the field of possible chemical species. These questions are based on the known relative responses of the sensors. For example, suppose two sensors, A and B, respond to two analytes, A' and B', but A has a stronger response to A' and similarly B responds more strongly to B'. On their own, A or B could not distinguish between A' and B'. However, a simple decision tree can be initiated when a response is detected, with a simple question: did A exhibit a larger response than B? If yes, the chemical species detected is A'. If no, the chemical species detected is B'. The questions can be formulated either algorithmically by a computer or by hand. An example of a decision tree from the sensor data is presented in FIG. 11 is shown in FIG. 12.

In another embodiment, the algorithm may be a genetic algorithm. In still another embodiment, the algorithm may be a neural network. The training set used can be either real or simulated data.

In one embodiment, the algorithm may be a parameterization of the sensor responses. The response of each sensor can be modeled by the Langmuir Equation:

$$R_i\left(k_{i,j},p_j\right)\propto\frac{k_{i,j}p_j}{1+k_{i,j}p_j}+C$$

where $R_i$ is the response of the i-th sensor, $k_{i,j}$ is the adsorption coefficient of the j-th analyte on the i-th sensor material, $p_j$ is the partial pressure of the j-th analyte, and C is a constant. Each $R_i$ is measured and the constants $k_{i,j}$ are known and stored in a library. Then, the total system response to an analyte, $\Delta$, can be described as:

$$\Delta_j=\Delta_j\left(R_1,R_2,\text{K},R_n,p_j\right)$$

When a chemical species is detected, the relative sensor response will be measured and compared to the training set (the $\Delta$'s). A regression or algorithm (e.g., k-nearest neighbor, least squares approximation) will be used to determine which cataloged species yields the responses closest to those that were measured. Restated, each analyte will produce a unique function of sensor responses that can be used to partition the solution space. A simple (non-limiting) example is shown in FIG. 13. In FIG. 13, the sensor responses for two materials and two analytes at various concentrations are shown. The sensor responses for each analyte can be described by unique linear equations. Suppose an unknown is detected that yields sensor responses given by the star. By using an algorithm, a determination is made that the unknown is more likely to be n-methyl-phenethylamine than phenethylamine, since $d_{n\text{-}m\text{-}PEA} < d_{PEA}$. In FIG. 13, the sensor response, $R_{resp}$, was used, but any of the aforementioned responses, or those not explicitly included, could be used instead.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the scope of the invention. Accordingly, it is not intended that the invention be limited, except as by the claims set forth below.

## Claims

1. A multimode gas sensor platform (100), comprising:

an array of electrode pairs (106) oriented on a substrate (102), wherein individual electrode pairs (106) are separately addressable; and

a plurality of detection zones (104), each detection zone (104) comprising at least one set of individual electrode pairs (106) within the array, said at least one set of individual electrode pairs (106) having organic nanofibers (108) uniformly deposited thereon, said organic nanofibers being responsive to association with a corresponding target material, wherein each detection zone has a different nanofiber material,

**characterized in that** the associated nanofibers vary their electrical conductivity upon exposure to specific target materials and a source excitation light, and

further **characterized by** a housing having an inlet and an outlet, wherein the plurality of detection zones are positioned in the housing between the inlet and the outlet; and
a light source (216) configured to illuminate at least a first detection zone within the plurality of detection zones.

**2.** The multimode platform of claim 1, wherein the corresponding target material for each detection zone is independently one or more of explosive compounds, explosive byproducts, explosive precursors, and drugs.

**3.** The multimode platform of claim 1, wherein the organic nanofibers

a) form a porous film of entangled nanofibers;
b) have a diameter of about 10 nm to about 900 nm;
c) are individually selected from the group consisting of: carbazole-cornered, arylene-ethynylene tetracyclic macromolecules, indolocarbazole derivatives thereof, a substituted perylene tetracarboxylic diimide molecule, a substituted a 3,4,9,10-tetracarboxyl perylene molecule, and mixtures thereof; or
d) are individually selected from the group consisting of: carbazole-cornered, arylene-ethynylene tetracyclic macromolecules, indolocarbazole derivatives thereof, a substituted perylene tetracarboxylic diimide molecule, a substituted a 3,4,9,10-tetracarboxyl perylene molecule, and mixtures thereof and wherein the organic nanofibers are individually selected from the group consisting of: a 3,4,9,10-tetracarboxyl perylene compound having structure I:

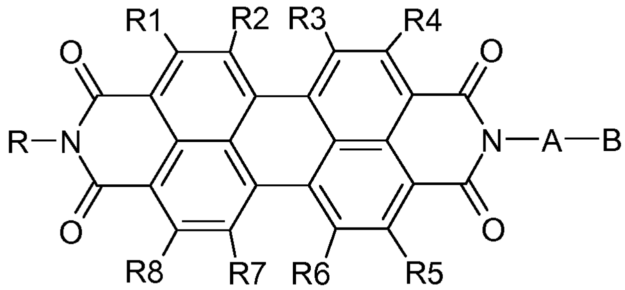

(I)

where R is a morphology control group, A is a linking group, B is a electron donor that is selective for transferring electrons to PTCDI backbone upon irradiation to make the resulting nanostructures conductive, and R1 through R8 are side groups; a alkyl-substituted, carbazole-cornered, arylene-ethynylene tetracyclic macromolecule of formula II:

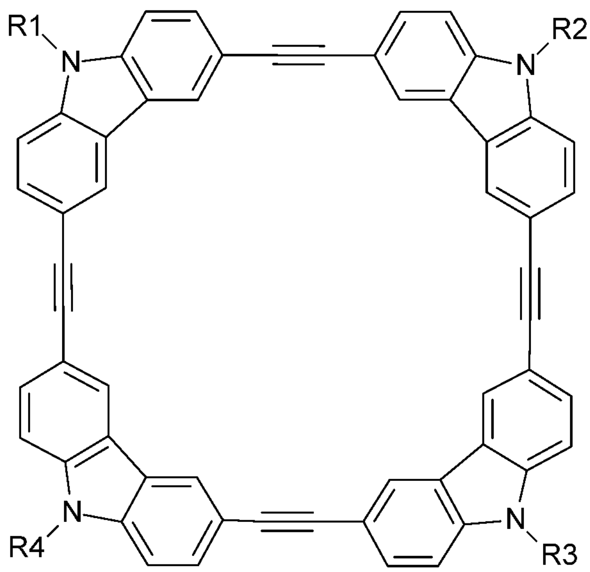

Formula II

wherein R1-R4 are alkyl groups and wherein at least some of the macromolecules are cofacially stacked; and

mixtures thereof.

4. The multimode platform of claim 1, wherein each of the detection zones are configured to detect an explosive compound selected from the group consisting of: trinitrotoluene (TNT); dinitrotoluene (DNT); 2,3-dimethyl-2,3-dinitrobutane (DMNB); 1,3,5-trinitroperhydro-1,3,5-triazine (RDX); pentaerythritol tetranitrate (PETN); Octahydro-1,3,5,7-tetranitro-1,3,5,7-tetrazocine (HMX); nitromethane; nitroglycerin; nitrocellulose; ethylene glycol dinitrate; dimethyl methylphosphonate; ammonium nitrate, urea nitrate; acetone peroxides; triacetone triperoxide (TATP); peroxyacetone; tri-cyclic acetone peroxide (TCAP); diacetone diperoxide (DADP); hexamethylene triperoxide diamine (HMTD); and composites or combinations thereof.

5. The multimode platform of claim 1, wherein the individual electrode pairs are interdigitated electrodes.

6. The multimode platform of claim 1, wherein

a) the plurality of detection zones includes at least one visual detection zone comprising organic nanofibers that fluoresce or have a visual color change when exposed to an explosive compound, an explosive byproduct, or an explosive precursor; or
b) the plurality of detection zones includes at least one visual detection zone comprising organic nanofibers that fluoresce or have a visual color change when exposed to an explosive compound, an explosive byproduct, or an explosive precursor and wherein the organic nanofibers are selected from the group consisting of: a linear carbazole oligomer, a porous hydrophilic material modified with a titanium oxo compound, and mixtures thereof; or
c) the plurality of detection zones includes at least one visual detection zone comprising organic nanofibers that fluoresce or have a visual color change when exposed to an explosive compound, an explosive byproduct, or an explosive precursor and wherein the organic nanofibers are selected from the group consisting of: a linear carbazole oligomer, a porous hydrophilic material modified with a titanium oxo compound, and mixtures thereof and wherein the organic nanofibers are individually selected from the group consisting of: a 3,4,9,10-tetracarboxyl perylene compound having the structure of formula III:

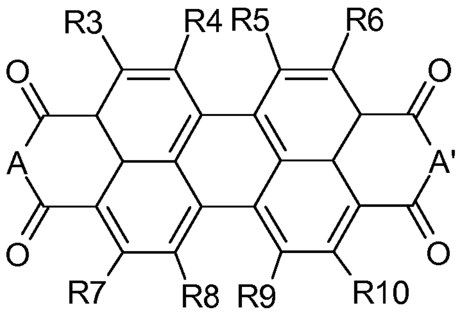

(III)

where A and A' are independently chosen from N-R1, N-R2, and O such that both A and A' are not O, and R1 through R10 are amine binding moieties, solubility enhancing groups, or hydrogen such that at least one of R1 through R10 is an amine binding moiety; a linear carbazole oligomer having the structure of formula IV:

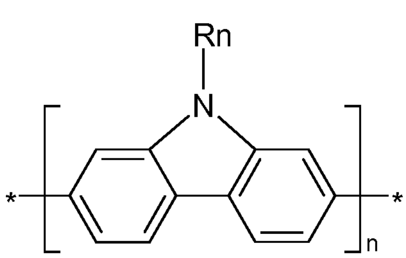

Formula IV

where n is 3 to 9, Rn are independently selected amine side groups, and at least one Rn is a C1 to C14 alkyl; a carbazole-cornered, arylene-ethynylene tetracyclic macromolecules of formula V:

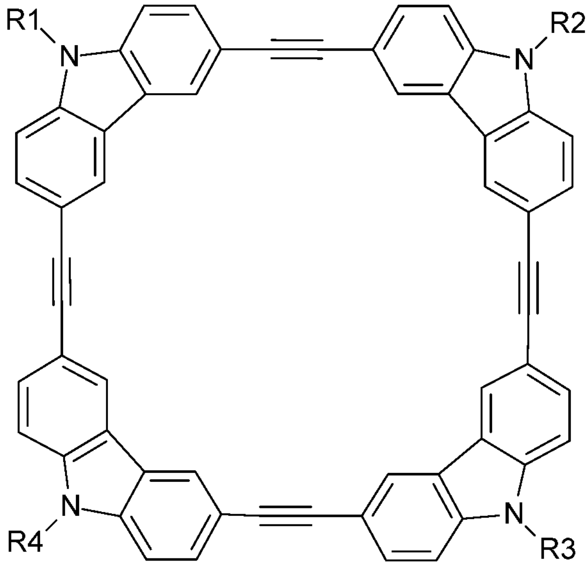

Formula V

wherein R1-R4 are alkyl-containing groups that facilitate cofacial stacking to form tubular morphology, and wherein at least some of the macromolecules are cofacially stacked; a porous hydrophilic material modified with a titanium oxo compound having the structure of formula VI:

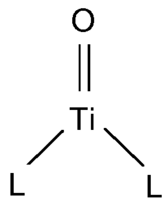

Formula VI

where L is a ligand, wherein the porous hydrophilic material is capable of detecting hydrogen peroxide vapor by complexing the titanium oxo compound with the hydrogen peroxide to provide a color change; and mixtures thereof.

**7.** The sensor of claim 1, wherein

a) the substrate of the multimode platform includes a plurality of holes allowing air flow from a top surface of the substrate to a bottom surface of the substrate; or
b) the substrate of the multimode platform includes a plurality of holes allowing air flow from a top surface of the substrate to a bottom surface of the substrate and wherein the multimode platform is isolated within the housing such that the air flow is forced through the plurality of holes.

**8.** The sensor of claim 1, wherein the plurality of detection zones include at least one visual detection zone comprising organic nanofibers that fluoresce or have a visual color change when exposed to the corresponding target material and the sensor further comprises a photodetector configured to detect the fluorescence or visual color change.

**9.** The sensor of claim 1, further comprising

a) a second light source that is configured to illuminate a second detection zone within the plurality of detection zones; and/or
b) a second light source that is configured to illuminate a second detection zone within the plurality of detection zones and wherein the first light source is an LED; and/or
c) a forced air mechanism adapted to move air across at least a portion of the plurality of detection zones; and/or
d) a microcontroller module adapted to measure a binding profile of a test sample and to correlate the binding profile with predetermined target compound binding profiles; and/or

e) an electrical interface adapted to connect to a computing device and transmit data from the plurality of detection zones to the computing device.

**10.** A method of detecting an explosive, comprising:
exposing the multimode platform of any one of claims 1 to 6 to a target sample; and measuring electrical responses of the organic nanofibers.

**11.** The method of claim 10, wherein the multimode platform further comprises a visual detection zone comprising organic nanofibers that fluoresce or have a visual color change when exposed to an explosive compound, an explosive byproduct, or an explosive precursor and measuring the fluorescence response or visual color change response of the organic nanofibers.

**12.** The method of claim 10, wherein the organic nanofibers are individually selected from the group consisting of: carbazole-cornered, arylene-ethynylene tetracyclic macromolecules, indolocarbazole derivatives thereof, a substituted perylene tetracarboxylic diimide molecule, a substituted a 3,4,9,10-tetracarboxyl perylene molecule, and mixtures thereof.

**13.** The method of claim 10, further comprising

a) measuring a characteristic based on the electrical responses selected from the group consisting of: a change in resistance, rate of response, rate of recovery, and reversibility of binding; or
b) measuring a characteristic based on the electrical responses selected from the group consisting of: a change in resistance, rate of response, rate of recovery, and reversibility of binding and further comprising using an algorithm to identify the target sample using the characteristic; or
c) measuring a characteristic based on the electrical responses selected from the group consisting of: a change in resistance, rate of response, rate of recovery, and reversibility of binding and further comprising using an algorithm to identify the target sample using the characteristic and wherein the algorithm is a parameterization of the sensor responses using the Langmuir Equation:

$$R_i\left(k_{i,j}, p_j\right) \propto \frac{k_{i,j} p_j}{1 + k_{i,j} p_j} + C$$

where $R_i$ is the response of the i-th sensor, $k_{i,j}$ is the adsorption coefficient of the j-th analyte on the i-th sensor material, $p_j$ is the partial pressure of the j-th analyte, and C is a constant, where each $R_i$ is measured and the constants $k_{i,j}$ are known and stored in a library; or d) measuring a characteristic based on the electrical responses selected from the group consisting of: a change in resistance, rate of response, rate of recovery, and reversibility of binding and further comprising using an algorithm to identify the target sample using the characteristic and wherein the algorithm is at least one of a decision tree, a genetic algorithm, and a neural network.

## Patentansprüche

**1.** Multimodale Gassensorplattform (100), umfassend:

ein Array von Elektrodenpaaren (106), die auf einem Substrat (102) orientiert sind, worin individuelle Elektrodenpaare (106) separat ansteuerbar sind; und
eine Mehrzahl von Nachweiszonen (104), wobei jede Nachweiszone (104) mindestens einen Satz von individuellen Elektrodenpaaren (106) innerhalb des Arrays umfasst, wobei der mindestens eine Satz von individuellen Elektrodenpaaren (106) organische Nanofasern (108), die einheitlich darauf abgelagert sind, aufweist, wobei die organischen Nanofasern auf Zuordnung zu einem entsprechenden Targetmaterial ansprechen, worin jede Nachweiszone ein anderes Nanofasermaterial aufweist, **dadurch gekennzeichnet, dass** die zugeordneten Nanofasern ihre elektrische Leitfähigkeit bei Exposition an spezifische Targetmaterialien und ein Quellenanregungslicht verändern, und ferner **gekennzeichnet durch**
ein Gehäuse mit einem Einlass und einem Auslass, worin die Mehrzahl von Nachweiszonen in dem Gehäuse zwischen dem Einlass und dem Auslass positioniert sind; und
eine Lichtquelle (216), die konfiguriert ist, um mindestens eine erste Nachweiszone innerhalb der Mehrzahl von

Nachweiszonen zu beleuchten.

2. Multimodale Plattform nach Anspruch 1, worin das entsprechende Targetmaterial für jede Nachweiszone unabhängig einer/eine/eines oder mehrere von explosiven Verbindungen, explosiven Nebenprodukten, explosiven Vorläufern und Arzneimitteln ist.

3. Multimodale Plattform nach Anspruch 1, worin die organischen Nanofasern

a) einen porösen Film von verschränkten Nanofasern bilden;
b) einen Durchmesser von etwa 10 nm bis etwa 900 nm aufweisen;
c) individuell ausgewählt sind aus der Gruppe bestehend aus: Carbazol-geeckten, tetrazyklischen Arylen-Ethynylen-Makromolekülen, Indolocarbazol-Derivaten davon, einem substituierten Perylen-Tetracarboxyl-Diimid-Molekül, einem substituierten $\alpha$-3,4,9,10-Tetracarboxylperylen-Molekül und Gemischen davon; oder
d) individuell ausgewählt sind aus der Gruppe bestehend aus: Carbazol-geeckten, tetrazyklischen Arylen-Ethynylen-Makromolekülen, Indolocarbazol-Derivaten davon, einem substituierten Perylen-Tetracarboxyl-Diimid-Molekül, einem substituierten $\alpha$-3,4,9,10-Tetracarboxylperylen-Molekül und Gemischen davon und worin die organischen Nanofasern individuell ausgewählt sind aus der Gruppe bestehend aus: $\alpha$-3,4,9,10-Tetracarboxylperylen-Verbindung mit der Struktur I:

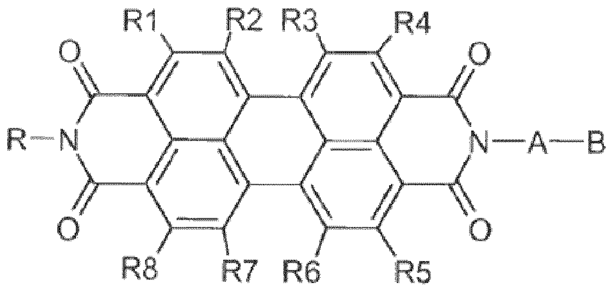

(I)

wobei R eine Morphologie-Kontrollgruppe ist, A eine verbindende Gruppe ist, B ein Elektronendonator ist, der selektiv beim Übertragen von Elektronen auf das PTCDI-Backbone bei Bestrahlung ist, um die resultierenden Nanostrukturen leitfähig zu machen, und R1 bis R8 Seitengruppen sind; $\alpha$-alkylsubstituiertem, Carbazol-geecktem, tetrazyklischem Arylen-Ethynylen-Makromolekül der Formel II:

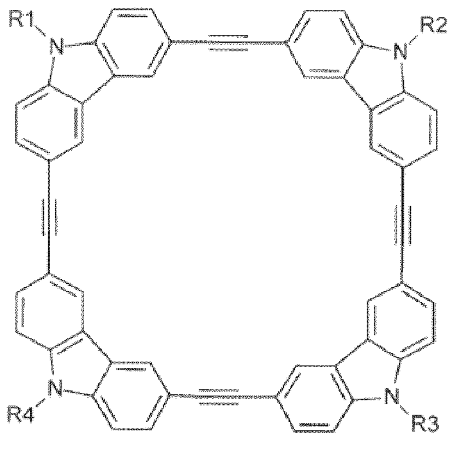

Formel II

worin R1-R4 Alkylgruppen sind und worin mindestens einige der Makromoleküle kofazial gestapelt sind; und Gemischen davon.

4. Multimodale Plattform nach Anspruch 1, worin jede der Nachweiszonen konfiguriert ist, um eine explosive Verbindung nachzuweisen, die ausgewählt ist aus der Gruppe bestehend aus:
Trinitrotoluol (TNT); Dinitrotoluol (DNT); 2,3-Dimethyl-2,3-dinitrobutan (DMNB); 1,3,5-Trinitroperhydro-1,3,5-triazin

(RDX); Pentaerythritoltetranitrat (PETN); Octahydro-1,3,5,7-tetranitro-1,3,5,7-tetrazocin (HMX); Nitromethan; Nitroglycerin; Nitrocellulose; Ethylenglycoldinitrat; Dimethylmethylphosphonat; Ammoniumnitrat, Harnstoffnitrat; Acetonperoxiden; Triacetontriperoxid (TATP); Peroxyaceton; trizyklischem Acetonperoxid (TCAP); Diacetondiperoxid (DADP); Hexamethylentriperoxiddiamin (HMTD); und Verbunden oder Kombinationen davon.

**5.** Multimodale Plattform nach Anspruch 1, worin die individuellen Elektrodenpaare interdigitierte Elektroden sind.

**6.** Multimodale Plattform nach Anspruch 1, worin

a) die Mehrzahl von Nachweiszonen mindestens eine visuelle Nachweiszone umfassend organische Nanofasern beinhaltet, die bei Exposition an eine explosive Verbindung, ein explosives Nebenprodukt oder einen explosiven Vorläufer fluoreszieren oder eine visuelle Farbveränderung aufweisen; oder
b) die Mehrzahl von Nachweiszonen mindestens eine visuelle Nachweiszone umfassend organische Nanofasern beinhaltet, die bei Exposition an eine explosive Verbindung, ein explosives Nebenprodukt oder einen explosiven Vorläufer fluoreszieren oder eine visuelle Farbveränderung aufweisen und worin die organischen Nanofasern ausgewählt sind aus der Gruppe bestehend aus: einem linearen Carbazol-Oligomer, einem porösen hydrophilen Material, das mit einer Titan-oxo-Verbindung modifiziert ist, und Gemischen davon; oder
c) die Mehrzahl von Nachweiszonen mindestens eine visuelle Nachweiszone umfassend organische Nanofasern beinhaltet, die bei Exposition an eine explosive Verbindung, ein explosives Nebenprodukt oder einen explosiven Vorläufer fluoreszieren oder eine visuelle Farbveränderung aufweisen und worin die organischen Nanofasern ausgewählt sind aus der Gruppe bestehend aus: einem linearen Carbazol-Oligomer, einem porösen hydrophilen Material, das mit einer Titan-oxo-Verbindung modifiziert ist, und Gemischen davon und worin die organischen Nanofasern individuell ausgewählt sind aus der Gruppe bestehend aus: α-3,4,9,10-Tetracarboxylperylen-Verbindung mit der Struktur der Formel III:

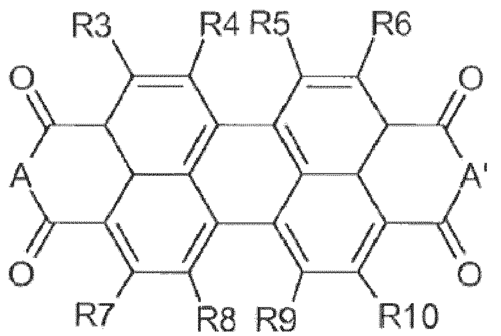

(III)

wobei A und A' unabhängig gewählt sind aus N-R1, N-R2 und O, so dass sowohl A als auch A' nicht O sind, und R1 bis R10 aminbindende Moietäten, löslichkeitsverbessernde Gruppen oder Wasserstoff sind, so dass mindestens eine von R1 bis R10 eine aminbindende Moietät ist; einem linearen Carbazol-Oligomer mit der Struktur der Formel IV:

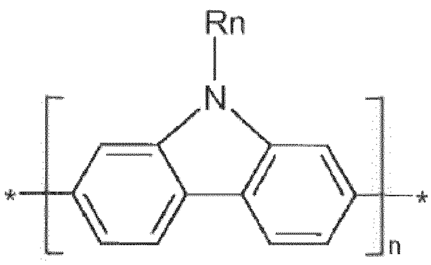

Formel IV

wobei n 3 bis 9 ist, Rn unabhängig gewählte Amin-Seitengruppen sind und mindestens eine Rn ein C1- bis C14-Alkyl ist; α-Carbazol-geeckten, tetrazyklischen Arylen-Ethynylen-Makromolekülen der Formel V:

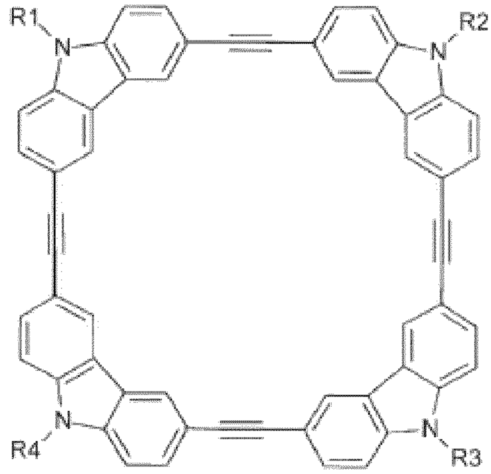

Formel V

worin R1-R4 alkylhaltige Gruppen sind, die kofaziales Stapeln ermöglichen, um eine röhrenförmige Morphologie zu bilden, und worin mindestens einige der Makromoleküle kofazial gestapelt sind; einem porösen hydrophilen Material, das mit einer Titan-oxo-Verbindung modifiziert ist, mit der Struktur der Formel VI:

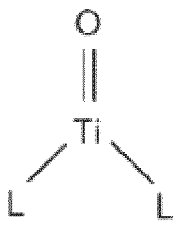

Formel VI

wobei L ein Ligand ist, worin das poröse hydrophile Material fähig ist, Wasserstoffsuperoxiddampf durch Komplexieren der Titan-oxo-Verbindung mit dem Wasserstoffsuperoxid nachzuweisen, um für eine Farbveränderung zu sorgen; und Gemischen davon.

**7.** Sensor nach Anspruch 1, worin

a) das Substrat der multimodalen Plattform eine Mehrzahl von Löchern beinhaltet, die Luftströmung von einer oberen Oberfläche des Substrats zu einer unteren Oberfläche des Substrats erlauben; oder
b) das Substrat der multimodalen Plattform eine Mehrzahl von Löchern beinhaltet, die Luftströmung von einer oberen Oberfläche des Substrats zu einer unteren Oberfläche des Substrats erlauben, und worin die multimodale Plattform innerhalb des Gehäuses isoliert ist, so dass die Luftströmung durch die Mehrzahl von Löchern hindurchgezwungen wird.

**8.** Sensor nach Anspruch 1, worin die Mehrzahl von Nachweiszonen mindestens eine visuelle Nachweiszone umfassend organische Nanofasern beinhaltet, die bei Exposition an das entsprechende Targetmaterial fluoreszieren oder eine visuelle Farbveränderung aufweisen, und der Sensor ferner einen Fotodetektor umfasst, der konfiguriert ist, um die Fluoreszenz oder visuelle Farbveränderung nachzuweisen.

**9.** Sensor nach Anspruch 1, ferner umfassend

a) eine zweite Lichtquelle, die konfiguriert ist, um eine zweite Nachweiszone innerhalb der Mehrzahl von Nachweiszonen zu beleuchten; und/oder
b) eine zweite Lichtquelle, die konfiguriert ist, um eine zweite Nachweiszone innerhalb der Mehrzahl von Nachweiszonen zu beleuchten, und worin die erste Lichtquelle eine LED ist; und/oder
c) einen Zwangsluftmechanismus, der ausgelegt ist, um Luft über mindestens einen Abschnitt der Mehrzahl von Nachweiszonen zu bewegen; und/oder

d) ein Microcontroller-Modul, das ausgelegt ist, um ein Bindungsprofil einer Testprobe zu messen und um das Bindungsprofil mit vorbestimmten Targetverbindungs-Bindungsprofilen zu korrelieren; und/oder
e) eine elektrische Schnittstelle, die ausgelegt ist, um an eine Rechenvorrichtung angeschlossen zu werden und Daten aus der Mehrzahl von Nachweiszonen an die Rechenvorrichtung zu übertragen.

**10.** Verfahren für den Nachweis eines Sprengstoffs, umfassend:
Aussetzen der multimodalen Plattform nach einem der Ansprüche 1 bis 6 an eine Targetprobe; und Messen elektrischer Reaktionen der organischen Nanofasern.

**11.** Verfahren nach Anspruch 10, worin die multimodale Plattform ferner eine visuelle Nachweiszone umfassend organische Nanofasern umfasst, die bei Exposition an eine explosive Verbindung, ein explosives Nebenprodukt oder einen explosiven Vorläufer fluoreszieren oder eine visuelle Farbveränderung aufweisen, und Messen der Fluoreszenzreaktion oder visuellen Farbveränderungsreaktion der organischen Nanofasern.

**12.** Verfahren nach Anspruch 10, worin die organischen Nanofasern individuell ausgewählt sind aus der Gruppe bestehend aus: Carbazol-geeckten, tetrazyklischen Arylen-Ethynylen-Makromolekülen, Indolocarbazol-Derivaten davon, einem substituierten Perylen-Tetracarboxyl-Diimid-Molekül, einem substituierten $\alpha$-3,4,9,10-Tetracarboxylperylen-Molekül und Gemischen davon.

**13.** Verfahren nach Anspruch 10, ferner umfassend

a) Messen eines Merkmals auf Basis der elektrischen Reaktionen, die ausgewählt sind aus der Gruppe bestehend aus: einer Veränderung des Widerstands, Reaktionsrate, Erholungsrate und Bindungsreversibilität; oder
b) Messen eines Merkmals auf Basis der elektrischen Reaktionen, die ausgewählt sind aus der Gruppe bestehend aus: einer Veränderung des Widerstands, Reaktionsrate, Erholungsrate und Bindungsreversibilität und ferner umfassend Verwendung eines Algorithmus zum Identifizieren der Targetprobe mithilfe des Merkmals; oder
c) Messen eines Merkmals auf Basis der elektrischen Reaktionen, die ausgewählt sind aus der Gruppe bestehend aus: einer Veränderung des Widerstands, Reaktionsrate, Erholungsrate und Bindungsreversibilität und ferner umfassend Verwendung eines Algorithmus zum Identifizieren der Targetprobe mithilfe des Merkmals und worin der Algorithmus eine Parametrierung der Sensorreaktionen mithilfe der Langmuir-Gleichung ist:

$$R_i\left(k_{i,j}, p_j\right) \propto \frac{k_{i,j} p_j}{1 + k_{i,j} p_j} + C$$

wobei $R_i$ die Reaktion des i-ten Sensors ist, $k_{i,j}$ der Adsorptionskoeffizient des j-ten Analyten auf dem i-ten Sensormaterial ist, $p_j$ der partielle Druck des j-ten Analyten ist und C eine Konstante ist, wobei jede $R_i$ gemessen wird und die Konstanten $k_{i,j}$ bekannt und in einer Bibliothek gespeichert sind; oder d) Messen eines Merkmals auf Basis der elektrischen Reaktionen, die ausgewählt sind aus der Gruppe bestehend aus: einer Veränderung des Widerstands, Reaktionsrate, Erholungsrate und Bindungsreversibilität und ferner umfassend Verwendung eines Algorithmus zum Identifizieren der Targetprobe mithilfe des Merkmals und worin der Algorithmus mindestens eines von einem Entscheidungsbaum, einem genetischen Algorithmus und einem neuronalen Netzwerk ist.

**Revendications**

**1.** Une plateforme de capteur de gaz multimode (100), comprenant :

un réseau de paires d'électrodes individuelles (106) orientées sur un substrat (102), dans laquelle des paires d'électrodes individuelles (106) peuvent être adressées séparément ; et
une pluralité de zones de détection (104), chaque zone de détection (104) comprenant un ou plusieurs ensembles de paires d'électrodes individuelles (106) dans le réseau, ledit ou lesdits ensembles de paires d'électrodes individuelles (106) ayant des nanofibres organiques (108) déposées uniformément sur celles-ci, lesdites nanofibres organiques étant sensibles à l'association avec un matériau cible correspondant, dans laquelle chaque zone de détection a un matériau de nanofibre différent, **caractérisé en ce que** les nanofibres associées varient

leur conductivité électrique lors de leur exposition à des matériaux cibles spécifiques et à une source de lumière d'excitation, et **caractérisé en outre par**
un logement ayant une entrée et une sortie, dans laquelle la pluralité de zones de détection sont positionnées dans le logement entre l'entrée et la sortie ; et
une source de lumière (216) configurée pour éclairer au moins une première zone de détection dans la pluralité de zones de détection.

**2.** La plateforme multimode selon la revendication 1, dans laquelle le matériau cible correspondant de chaque zone de détection est indépendamment un ou plusieurs parmi des composés explosifs, des sous-produits explosifs, des précurseurs explosifs et des drogues.

**3.** La plateforme multimode selon la revendication 1, dans laquelle les nanofibres organiques

a) forment un film poreux de nanofibres enchevêtrées ;
b) ont un diamètre compris entre environ 10 nm et environ 900 nm ;
c) sont sélectionnées individuellement dans le groupe constitué par : des macromolécules tétracycliques d'arylène-éthynylène à carbazole en coin, des dérivés d'indolocarbazole de celles-ci, une molécule de diimide tétracarboxylique de pérylène substituée, une molécule de diimide de pérylène α-3,4,9,10-tétracarboxylique substituée, et des mélanges de ces derniers ; ou
d) sont sélectionnées individuellement dans le groupe constitué par : des macromolécules tétracycliques d'arylène-éthynylène à carbazole en coin, des dérivés d'indolocarbazole de celles-ci, une molécule de diimide tétracarboxylique de pérylène substituée, une molécule de pérylène α-3,4,9,10-tétracarboxylique substituée, et des mélanges de ces derniers et dans laquelle les nanofibres organiques sont sélectionnées individuellement dans le groupe constitué par : un composé de pérylène α-3,4,9,10-tétracarboxylique ayant la structure I :

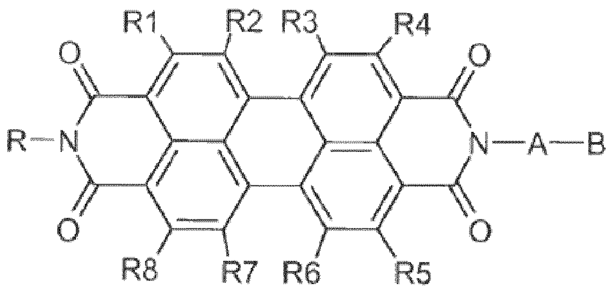

(I)

où R est un groupe témoin de morphologie, A est un groupe de liaison, B est un donneur d'électrons qui est sélectif pour transférer des électrons à un squelette PTCDI lors de l'irradiation pour rendre conductrices les nanostructures résultantes, et R1 à R8 sont des groupes latéraux ; une macromolécule tétracyclique d'arylène-éthynylène à carbazole en coin, substituée par un alkyle, de la formule II :

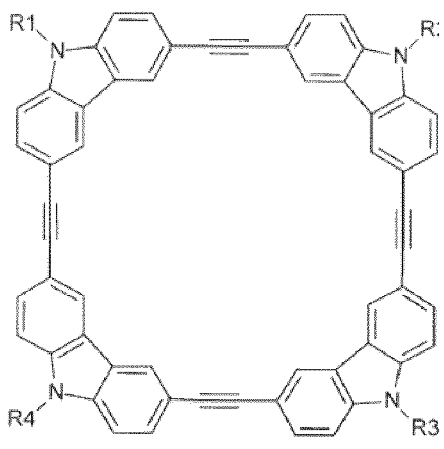

Formule II

dans laquelle R1-R4 sont des groupes alkyle et dans laquelle au moins certaines des macromolécules sont

empilées de façon cofaciale ; et des mélanges de ces derniers.

**4.** La plateforme multimode selon la revendication 1, dans laquelle chacune des zones de détection est configurée pour détecter un composé explosif sélectionné dans le groupe constitué par : trinitrotoluène (TNT) ; dinitrotoluène (DNT) ; 2,3-diméthyl-2,3-dinitrobutane (DMNB) ; 1,3,5-trinitroperhydro-1,3,5-triazine (RDX) ; tétranitrate de pentaérythritol (PETN) ; octahydro-1,3,5,7-tétranitro-1,3,5,7-tétrazocine (HMX) ; nitrométhane ; nitroglycérine ; nitrocellulose ; dinitrate d'éthylène glycol ; méthylphosphonate de diméthyle ; nitrate d'ammonium, nitrate d'urée ; peroxydes d'acétone ; triperoxyde de triacétone (TATP) ; peroxyacétone ; peroxyde d'acétone tricyclique (TCAP) ; diperoxyde de diacétone (DADP) ; triperoxyde d'hexaméthylène diamine (HMTD) ; et des composites ou des combinaisons de ces derniers.

**5.** La plateforme multimode selon la revendication 1, dans laquelle les paires d'électrodes individuelles sont des électrodes interdigitées.

**6.** La plateforme multimode selon la revendication 1, dans laquelle

a) la pluralité de zones de détection incluent une ou plusieurs zones de détection visibles comprenant des nanofibres organiques qui sont fluorescentes ou ont un changement de couleur visible lorsqu'elles sont exposées à un composé explosif, un sous-produit explosif ou un précurseur explosif ; ou
b) la pluralité de zones de détection incluent une ou plusieurs zones de détection visibles comprenant des nanofibres organiques qui sont fluorescentes ou ont un changement de couleur visible lorsqu'elles sont exposées à un composé explosif, un sous-produit explosif ou un précurseur explosif et dans laquelle les nanofibres organiques sont sélectionnées dans le groupe constitué par : un oligomère carbazole linéaire, un matériau hydrophile poreux modifié par un composé oxo du titane, et des mélanges de ces derniers ; ou
c) la pluralité de zones de détection incluent une ou plusieurs zones de détection visibles comprenant des nanofibres organiques qui sont fluorescentes ou ont un changement de couleur visible lorsqu'elles sont exposées à un composé explosif, un sous-produit explosif ou un précurseur explosif et dans laquelle les nanofibres organiques sont sélectionnées dans le groupe constitué par ; un oligomère carbazole linéaire, un matériau hydrophile poreux modifié par un composé oxo du titane, et des mélanges de ces derniers et dans laquelle les nanofibres organiques sont sélectionnées individuellement dans le groupe constitué par ; un composé de pérylène $\alpha$-3,4,9,10-tétracarboxylique ayant la structure de la formule III ;

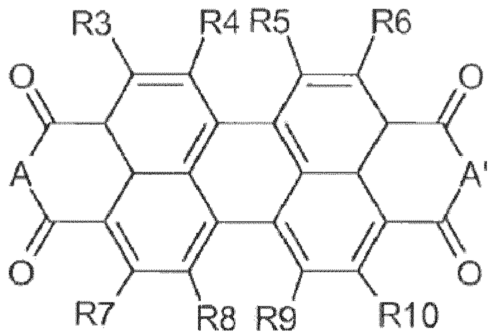

(III)

où A et A' sont choisis indépendamment parmi N-R1, N-R2 et O de telle sorte que A et A' ne sont pas O, et que R1 à R10 sont des fractions de liaison d'amine, des groupes améliorant la solubilité ou un hydrogène de telle sorte qu'au moins un R parmi R1 à R10 est une fraction de liaison d'amine ; un oligomère carbazole linéaire ayant la structure de la formule IV :

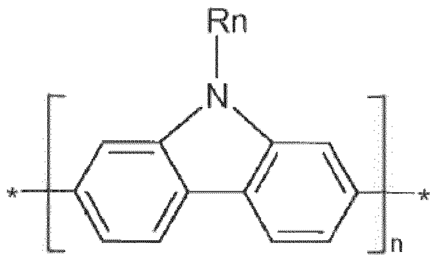

Formula IV

où n est 3 à 9, Rn sont des groupes latéraux d'amines sélectionnés indépendamment, et au moins un Rn est un alkyle en C1 à C14 ; une macromolécule tétracyclique d'arylène-éthynylène à carbazole en coin, de la formule V :

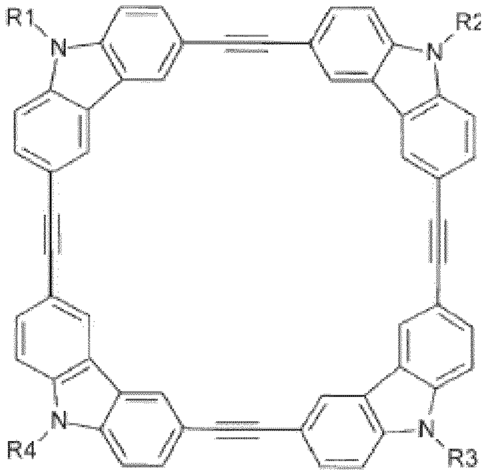

Formule V

dans laquelle R1-R4 sont des groupes contenant un alkyle qui facilitent un empilement cofacial pour former une morphologie tubulaire, et dans laquelle au moins certaines des macromolécules sont empilées de façon cofaciale ; un matériau hydrophile poreux modifié par un composé oxo du titane ayant la structure de la formule VI :

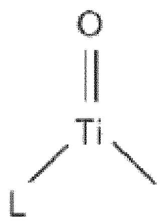

Formule VI

où L est un ligand, dans laquelle le matériau hydrophile poreux est capable de détecter une vapeur de peroxyde d'hydrogène en complexant le composé oxo du titane avec le peroxyde d'hydrogène pour fournir un changement de couleur ; et des mélanges de ces derniers.

**7.** Le capteur selon la revendication 1, dans lequel

a) le substrat de la plateforme multimode inclut une pluralité de trous permettant un écoulement d'air d'une surface supérieure du substrat à une surface inférieure du substrat ; ou
b) le substrat de la plateforme multimode inclut une pluralité de trous permettant un écoulement d'air d'une surface supérieure du substrat à une surface inférieure du substrat et dans lequel la plateforme multimode est isolée dans le logement de telle sorte que l'écoulement d'air est forcé à travers la pluralité de trous.

**8.** Le capteur selon la revendication 1, dans lequel la pluralité de zones de détection incluent une ou plusieurs zones de détection visibles comprenant des nanofibres organiques qui sont fluorescentes ou ont un changement de couleur visible lorsqu'elles sont exposées au matériau cible correspondant et le capteur comprend en outre un photodétecteur configuré pour détecter la fluorescence ou le changement de couleur visible.

**9.** Le capteur selon la revendication 1, comprenant en outre

a) une deuxième source de lumière qui est configurée pour éclairer une deuxième zone de détection dans la pluralité de zones de détection ; et/ou
b) une deuxième source de lumière qui est configurée pour éclairer une deuxième zone de détection dans la pluralité de zones de détection et dans lequel la première source de lumière est une LED ; et/ou
c) un mécanisme d'air forcé adapté pour déplacer l'air sur au moins une partie de la pluralité de zones de détection ; et/ou
d) un module de microcontrôleur adapté pour mesurer un profil de liaison d'un échantillon d'essai et pour corréler le profil de liaison avec des profils de liaison de composés de cibles prédéterminés ; et/ou
e) une interface électrique adaptée pour se connecter à un dispositif informatique et transmettre des données de la pluralité de zones de détection au dispositif informatique.

**10.** Un procédé de détection d'un explosif, consistant à :

exposer la plateforme multimode selon l'une quelconque des revendications 1 à 6 à un échantillon cible ; et mesurer des réponses électriques des nanofibres organiques.

**11.** Le procédé selon la revendication 10, dans lequel la plateforme multimode comprend en outre une zone de détection visible comprenant des nanofibres organiques qui sont fluorescentes ou ont un changement de couleur visible lorsqu'elles sont exposées à un composé explosif, un sous-produit explosif ou un précurseur explosif et mesure la réponse de fluorescence ou la réponse de changement de couleur visible des nanofibres organiques.

**12.** Le procédé selon la revendication 10, dans lequel les nanofibres organiques sont sélectionnées individuellement dans le groupe constitué par : des macromolécules tétracycliques d'arylène-éthynylène à carbazole en coin, des dérivés d'indolocarbazole de celles-ci, une molécule de diimide tétracarboxylique de pérylène substituée, une molécule de pérylène $\alpha$-3,4,9,10-tétracarboxylique substituée, et des mélanges de ces derniers.

**13.** Le procédé selon la revendication 10, consistant en outre à

a) mesurer une caractéristique sur la base des réponses électriques sélectionnées dans le groupe constitué par : un changement de la résistance, du taux de réponse, du taux de récupération et de la réversibilité de la liaison ; ou
b) mesurer une caractéristique sur la base des réponses électriques sélectionnées dans le groupe constitué par : un changement de la résistance, du taux de réponse, du taux de récupération et de la réversibilité de la liaison et consistant en outre à utiliser un algorithme pour identifier l'échantillon cible en utilisant la caractéristique ; ou
c) mesurer une caractéristique sur la base des réponses électriques, sélectionnée dans le groupe constitué par : un changement de la résistance, du taux de réponse, du taux de récupération et de la réversibilité de la liaison et consistant en outre à utiliser un algorithme pour identifier l'échantillon cible en utilisant la caractéristique et dans laquelle l'algorithme est un paramétrage des réponses du capteur en utilisant l'équation de Langmuir :

$$R_i\left(k_{i,j},p_j\right)\propto\frac{k_{i,j}p_j}{1+k_{i,j}p_j}+C$$

où $R_i$ est la réponse du i-ème capteur, $k_{i,j}$ est le coefficient d'adsorption du j-ème analyte sur le i-ème matériau du capteur, $p_j$ est la pression partielle du j-ème analyte, et C est une constante, où chaque $R_i$ est mesuré et les constantes $k_{i,j}$ sont connues et stockées dans une bibliothèque; ou
d) mesurer une caractéristique sur la base des réponses électriques, sélectionnée dans le groupe constitué par : un changement de la résistance, du taux de réponse, du taux de récupération et de la réversibilité de la liaison et consistant en outre à utiliser un algorithme pour identifier l'échantillon cible en utilisant la caractéristique

et dans laquelle l'algorithme est soit un arbre de décision, soit un algorithme génétique, soit un réseau neuronal.

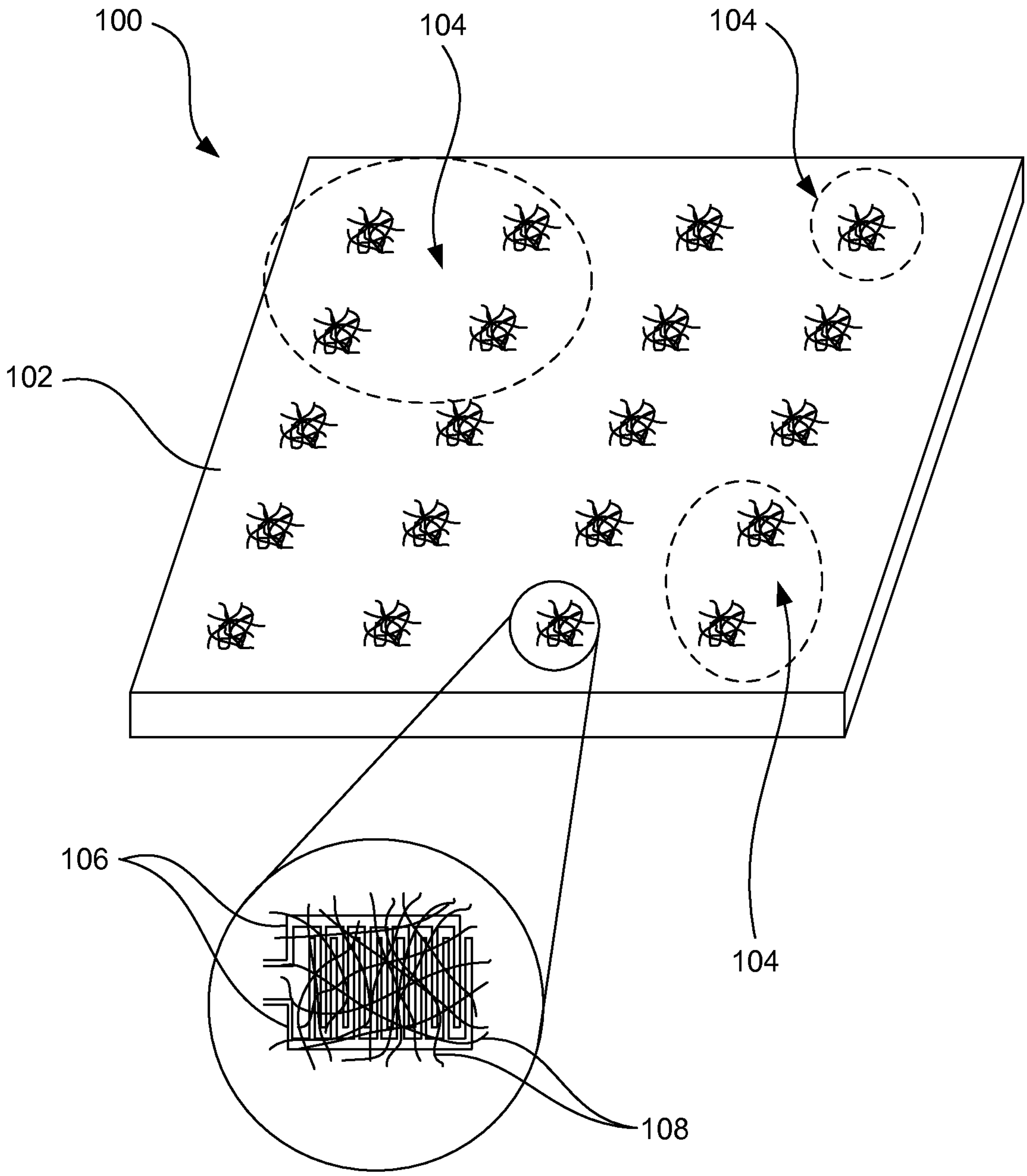


FIG. 1

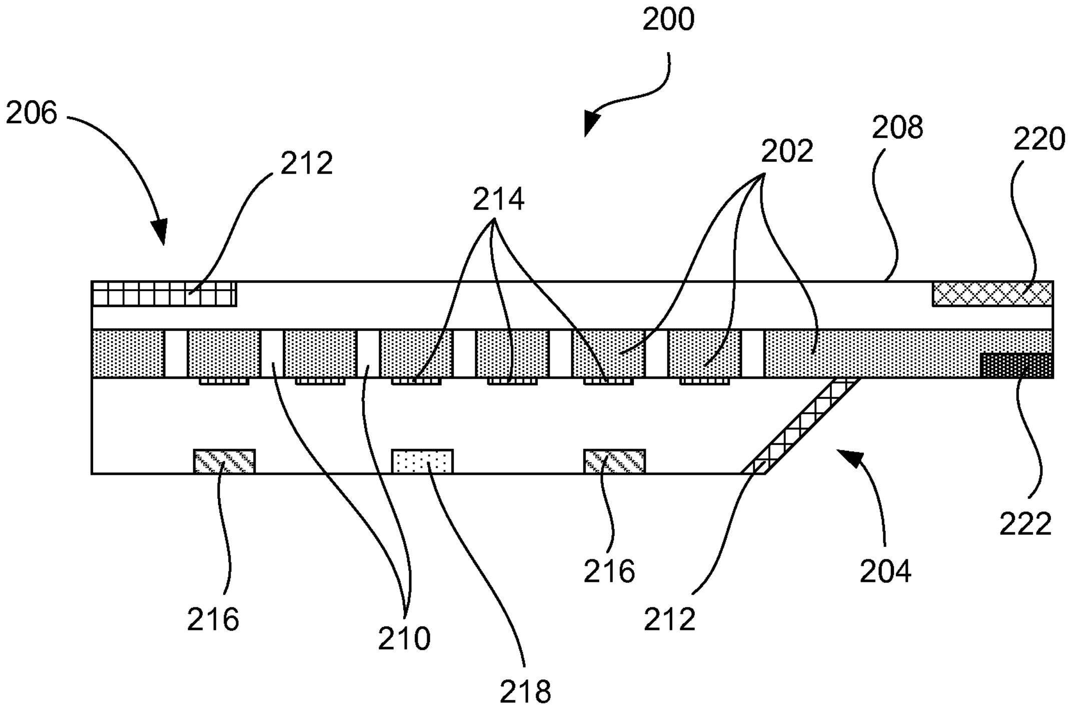


FIG. 2

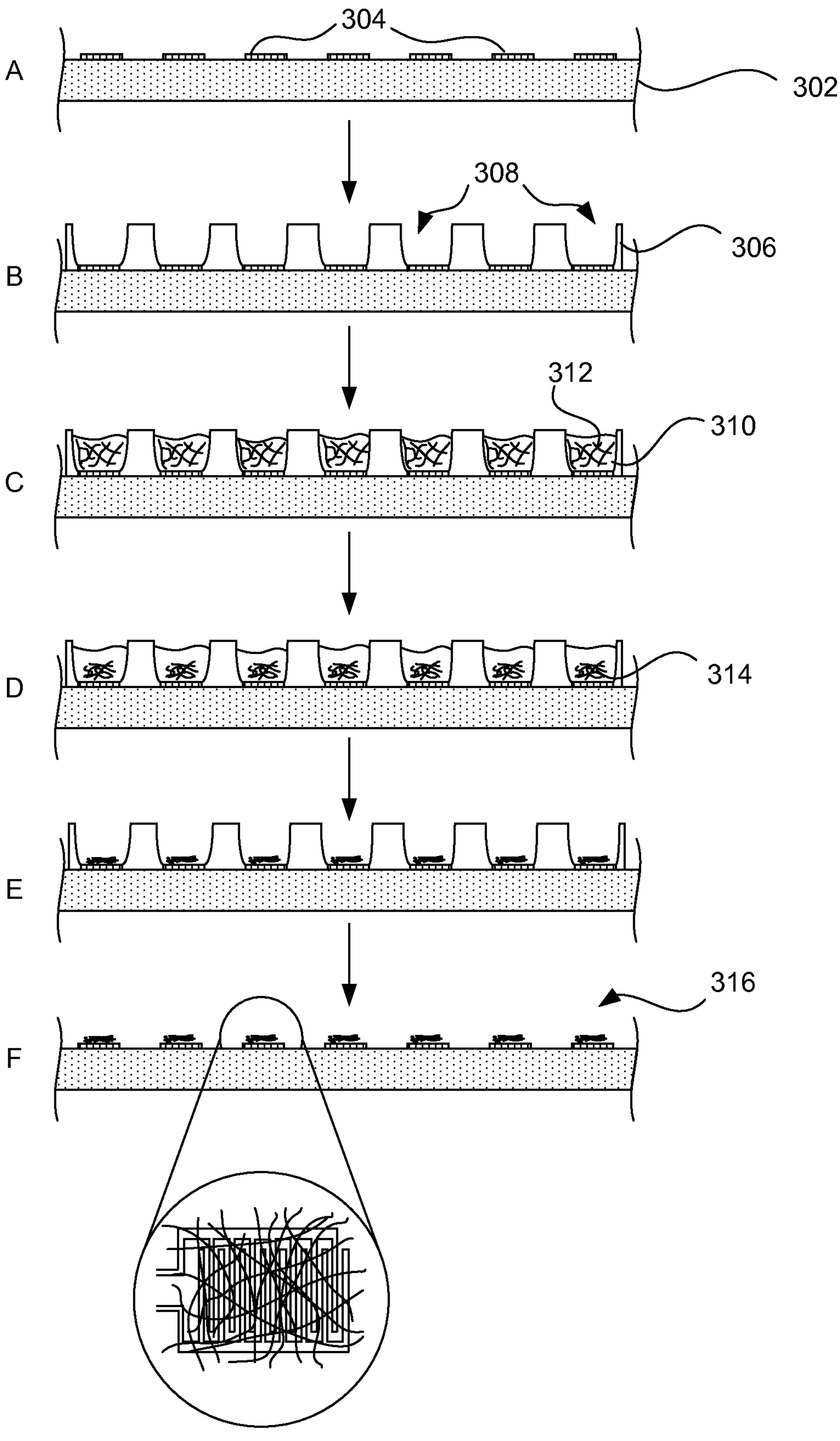


FIG. 3

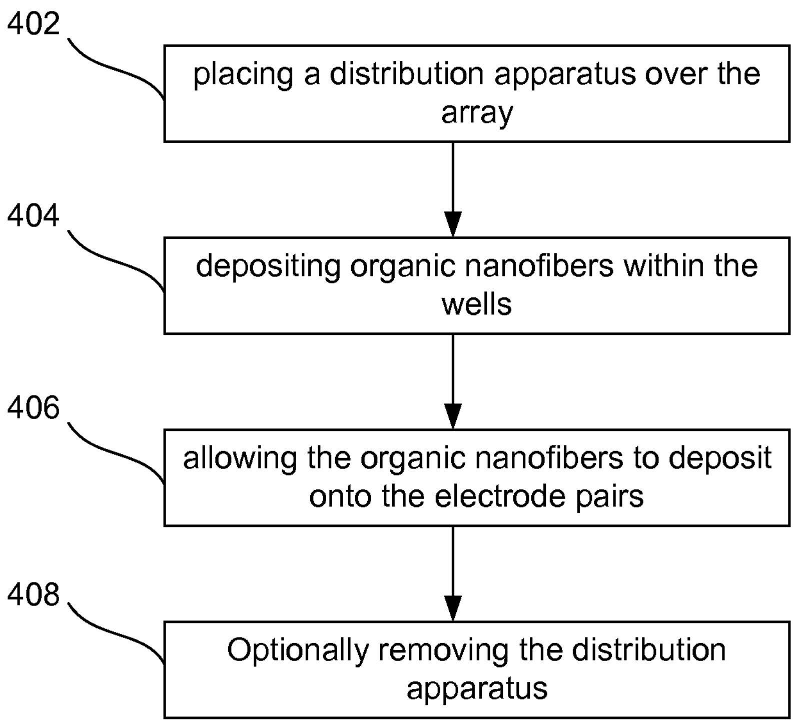
402
placing a distribution apparatus over the array
404
depositing organic nanofibers within the wells
406
allowing the organic nanofibers to deposit onto the electrode pairs
408
Optionally removing the distribution apparatus

FIG. 4

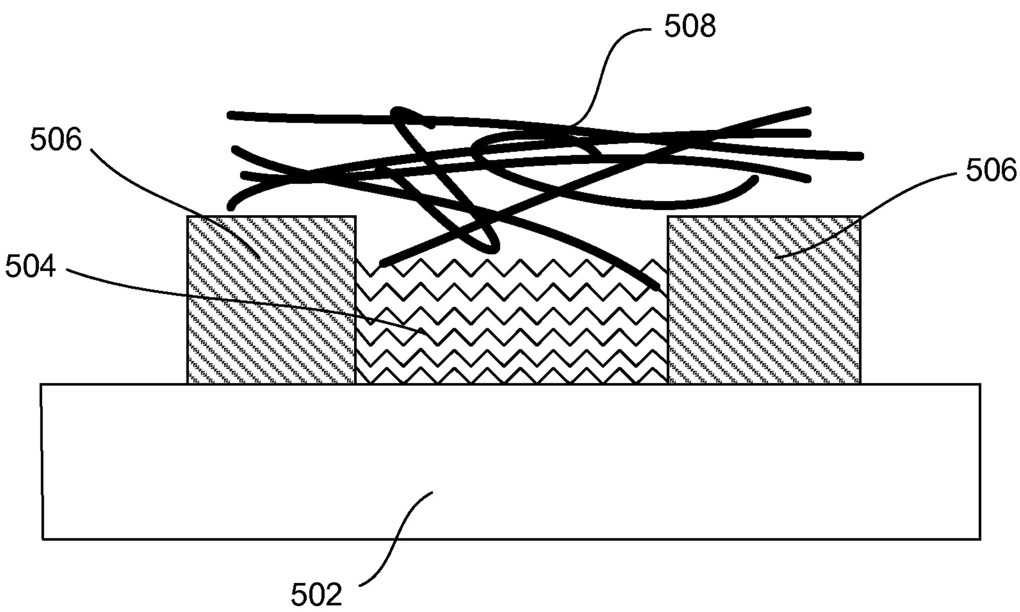
508
506
506
504
502

FIG. 5

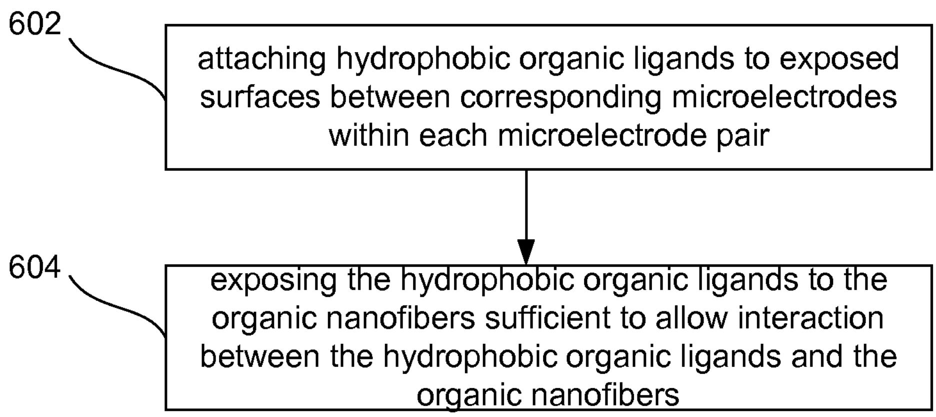
602
attaching hydrophobic organic ligands to exposed surfaces between corresponding microelectrodes within each microelectrode pair
604
exposing the hydrophobic organic ligands to the organic nanofibers sufficient to allow interaction between the hydrophobic organic ligands and the organic nanofibers

*FIG. 6*

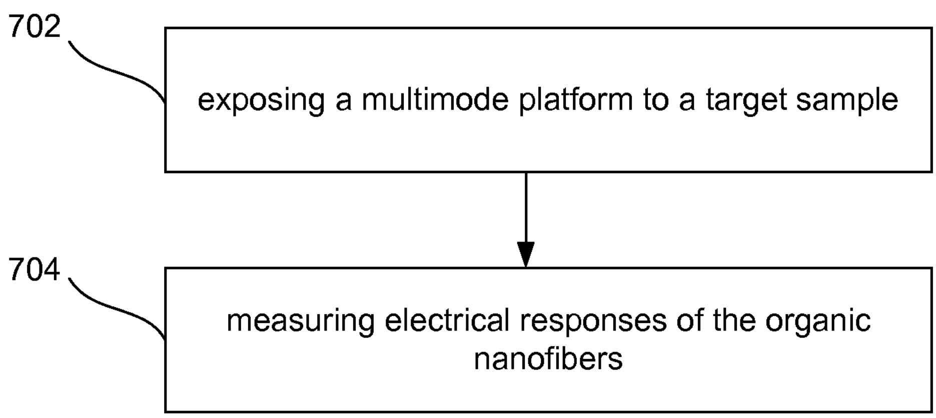
702
exposing a multimode platform to a target sample
704
measuring electrical responses of the organic nanofibers

*FIG. 7*

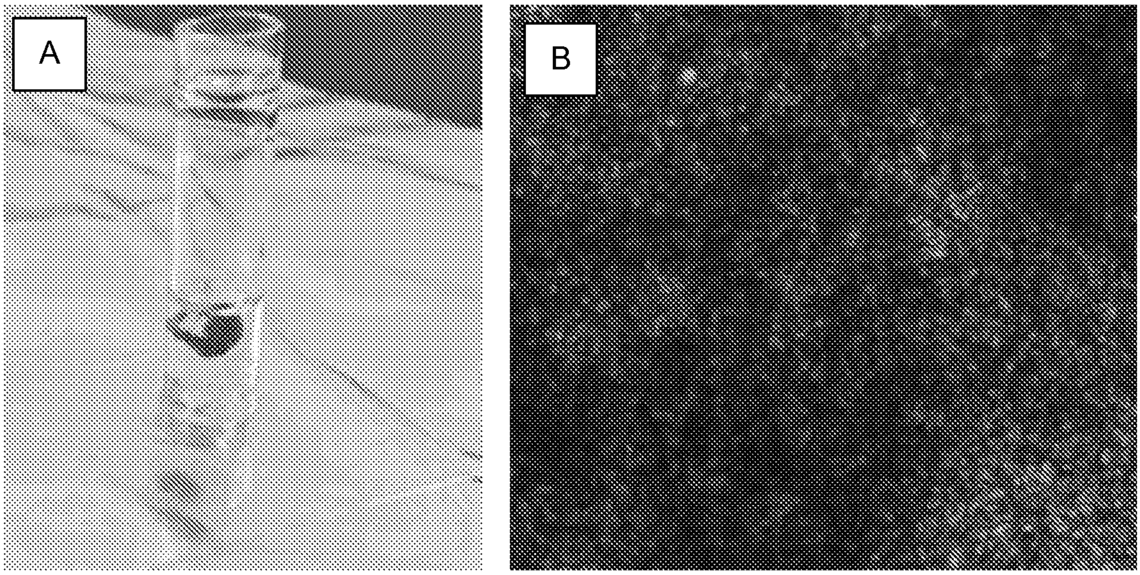


*FIG. 8*

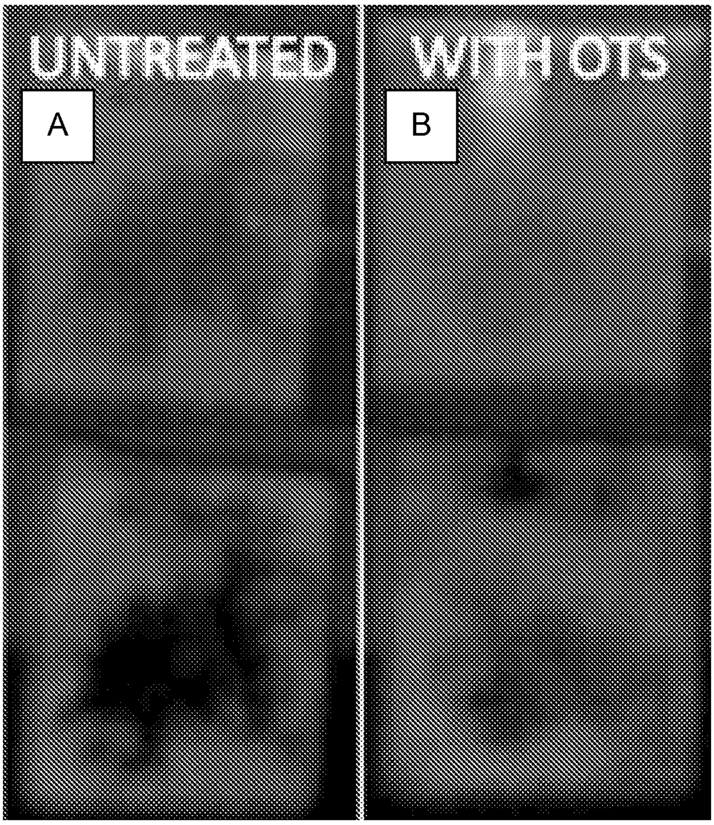


*FIG. 9*

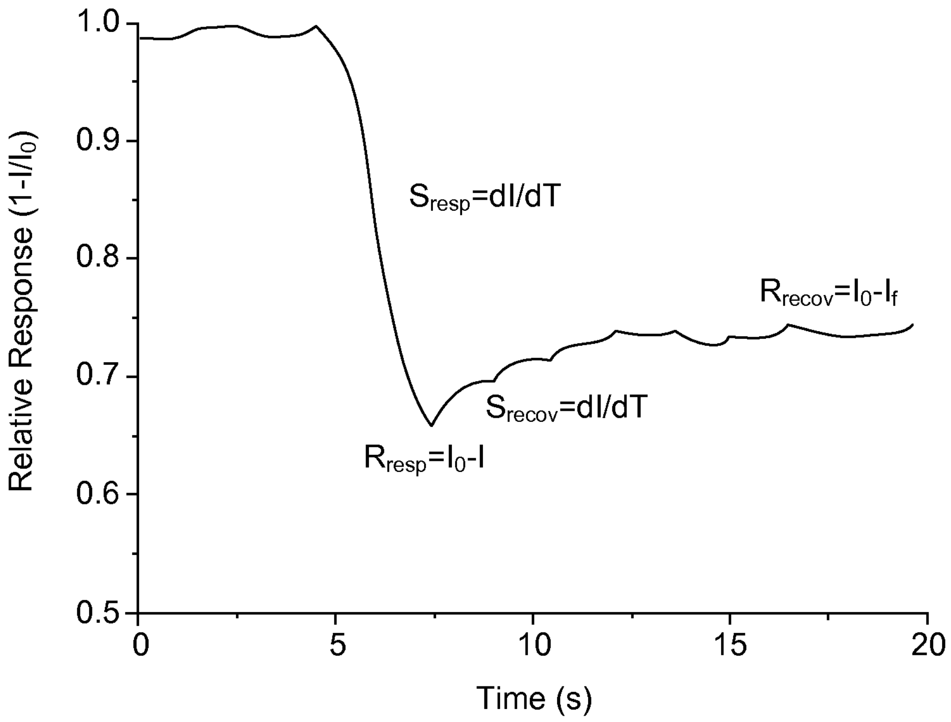
1.0
0.9
0.8
0.7
0.6
0.5
Relative Response (1-I/I0)
0
5
10
15
20
Time (s)
Sresp=dI/dT
Rrecov=I0-If
Srecov=dI/dT
Rresp=I0-I

*FIG. 10*

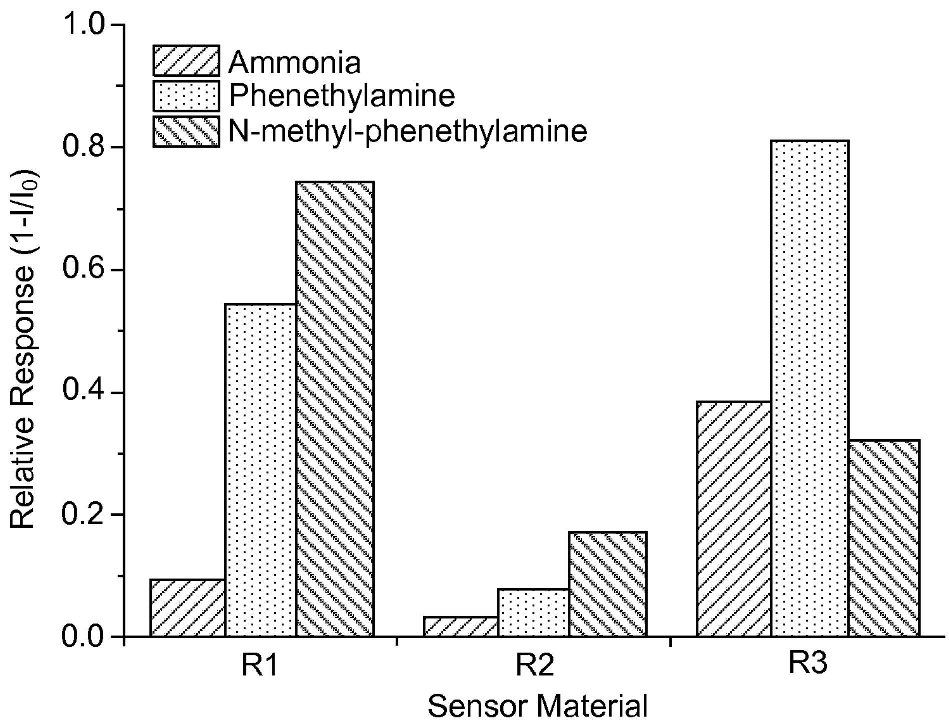
1.0
0.8
0.6
0.4
0.2
0.0
Relative Response (1-I/I0)
Ammonia
Phenethylamine
N-methyl-phenethylamine
R1
R2
R3
Sensor Material

*FIG. 11*

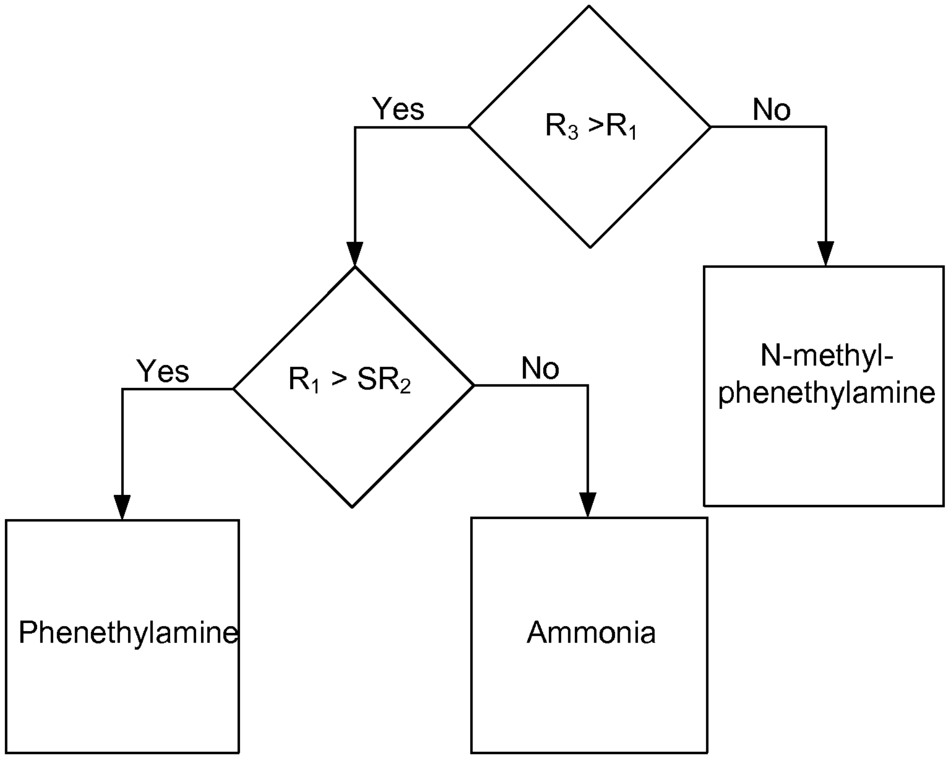
Yes
$R_3 > R_1$
No
Yes
$R_1 > SR_2$
No
N-methyl-phenethylamine
Phenethylamine
Ammonia

*FIG. 12*

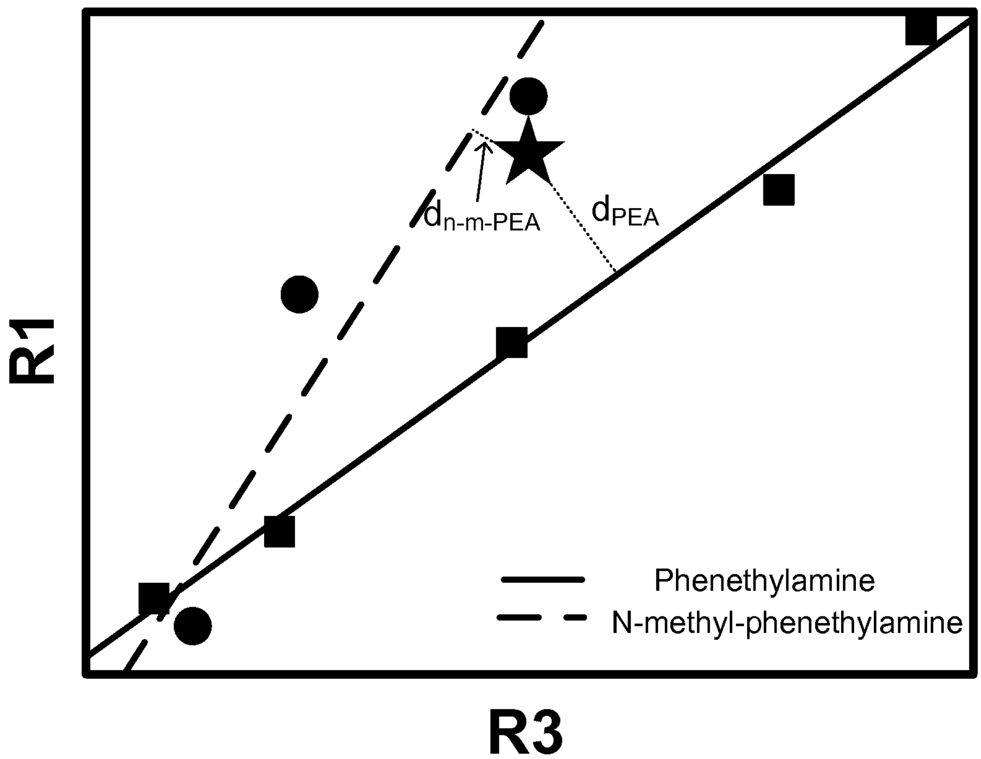
$d_{n\text{-}m\text{-}PEA}$
$d_{PEA}$
R1
R3
Phenethylamine
N-methyl-phenethylamine

*FIG. 13*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2008150556 A
- WO 2011119752 A
- WO 2011079296 A
- US 20100197039 A
- WO 2012047330 A
- WO 2013066458 A
- WO 2011100010 A


### Non-patent literature cited in the description


- **NADDO T et al.** *SENSORS AND ACTUATORS B,* 08 May 2008, vol. 134 (1), 287-291